(19) Europäisches Patentamt
European Patent Office
Office européen des brevets

(11) EP 1 967 522 A1

(12) EUROPEAN PATENT APPLICATION

(43) Date of publication:
**10.09.2008 Bulletin 2008/37**

(21) Application number: **07103677.6**

(22) Date of filing: **07.03.2007**

(51) Int Cl.:
***C07F 9/40*** *(2006.01)* ***A61K 31/662*** *(2006.01)*
***A61P 7/02*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **NOVO NORDISK A/S**
**2880 Bagsvaerd (DK)**

(72) Inventor: **The designation of the inventor has not yet been filed**

(74) Representative: **Nilsson, Karin Norvin**
**Novo Nordisk A/S,**
**Corporate Patents,**
**Novo Allé**
**2880 Bagsvaerd (DK)**

(54) **New blood coagulation factor inhibitors**

(57) The invention relates to novel compounds with formula (I) wherein $R^1$-$R^7$ are as herein defined useful as blood coagulation factor inhibitors. The compounds (I) may be used for treatment of thrombotic conditions or as stabilizers of liquid formulations of blood coagulation factors, in particular liquid formulations of FVIIA, Factor VII variants, or Factor VII derivatives.

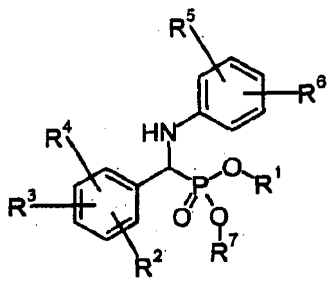

EP 1 967 522 A1

## Description

### FIELD OF THE INVENTION

The present invention relates to new inhibitors of the proteases involved in the blood coagulation cascade, and to use of said inhibitors as stabilizers of blood coagulation factors such as serine proteases and vitamin K-dependent polypeptides, as additives or formulation-aids for blood coagulation factors. In particular, the invention relates to stabilizing Factor VIIa or other Factor VII polypeptides against chemical and/or physical degradation, particularly in aqueous liquid compositions thereof.

### BACKGROUND OF THE INVENTION

The serine protease Factor VII (FVII) is one of the plasma glycoproteins involved in the blood coagulation process. FVII is mainly present in plasma as single-chain zymogen, and is cleaved by another protease (FXa), to give its two-chain, activated form, FVIIa.
The tissue factor/factor VIIa (TF/FVIIa) complex is the main trigger of thrombotic events. This complex is part of the extrinsic pathway of blood coagulation and mediates the activation of factors IX and X, ultimately leading to the generation of thrombin.
Factor VIIa has proven to be a valuable therapeutic agent for the treatment of haemophilia and bleeding. It is desirable to have administration forms of Factor VIIa suitable for both storage and for delivery. Ideally, the drug product is stored and administered as a liquid. Alternatively, the drug product is lyophilized, i.e. freeze-dried, and then reconstituted by adding a suitable diluent prior to patient use. It is desirable that the drug product has sufficient stability to enable long-term storage, e.g. for more than six months.
The decision whether to maintain the finished drug product as a liquid or to freeze-dry it is usually made on the basis of the stability of the protein drug in those forms. Protein stability can be affected, inter alia, by such factors as ionic strength, pH, temperature, repeated cycles of freeze/thaw, and exposure to shear forces. Active protein may be lost as a result of physical instabilities, e.g. via denaturation and/or aggregation (both soluble and insoluble aggregate formation), as well as chemical instabilities, including, for example, instability towards hydrolysis, deamidation and/or oxidation, to name just a few. Moreover, in the case of Factor VIIa, which is a serine protease, fragmentation due to autocatalysis may occur (autoproteolysis; enzymatic, self-catalyzed degradation). For a general review of the stability of protein pharmaceuticals, see, for example, Manning, et al., Pharmaceutical Research 6:903-918 (1989).
While the possible occurrence of protein instabilities is widely appreciated, it is generally very difficult to predict particular instability problems for a particular protein. Any of these instabilities can result in the formation of a protein by-product, or derivative, having reduced activity, increased toxicity and/or increased immunogenicity. Indeed, protein precipitation may lead to thrombosis, non-homogeneity of dosage form and amount, as well as clogged syringes. Furthermore, post-translational modifications such as, for example, gamma carboxylation of certain glutamic acid residues in the N-terminus and addition of carbohydrate side chains provide potential sites that may be susceptible to modification upon storage.
Thus, the safety and efficacy of any composition of a protein is directly related to its stability. Maintaining stability in a liquid form is generally a different task than maintaining stability in a lyophilized form because of highly increased potential for molecular motion and thereby increased probability of molecular interactions. Maintaining stability in a concentrated form is also a different task than the above, because of the propensity for aggregate formation at increased protein concentrations. Factor VIIa undergoes degradation by several pathways, especially aggregation (dimerisation/oligomerisation), oxidation and autolytic cleavage (clipping of the peptide backbone or "heavy chain degradation"). Furthermore, precipitation may occur.
Many of these processes can be slowed significantly by removal of water from the protein. However, the development of an aqueous composition for Factor VIIa has the advantages of eliminating reconstitution errors, thereby increasing dosing accuracy, as well as simplifying the use of the product clinically, thereby increasing patient compliance. Ideally, compositions of Factor VIIa should be stable for more than 6 months over a wide range of protein concentrations. This allows for flexibility in methods of administration. Generally, more highly concentrated forms allow for the administration of lower volumes, which is highly desirable from the patients' point of view. Liquid compositions can have many advantages over freeze-dried products with regard to ease of administration and use.
When developing a liquid composition, many factors are taken into consideration. Short-term, i.e. less than six months, liquid stability generally depends on avoiding gross structural changes, such as denaturation and aggregation. These processes are described in the literature for a number of proteins, and many examples of stabilizing agents exist. It is well-known that an agent effective in stabilizing one protein actually acts to destabilize another. Once the protein has been stabilized against gross structural changes, developing a liquid composition for long-term stability (e.g., greater than six months) depends on further stabilizing the protein from types of degradation specific to that protein. More specific

types of degradation may include, for example, disulfide bond scrambling, oxidation of certain residues, deamidation, cyclization. Although it is not always possible to pinpoint the individual degradation species, assays are developed to monitor subtle changes so as to monitor the ability of specific excipients to uniquely stabilize the protein of interest.

Today, the only commercially available, recombinantly produced FVII polypeptide composition is a freeze-dried Factor FVIIa product which is reconstituted before use; it contains a relatively low Factor VIIa concentration, e.g., about 0.6 mg/mL. A vial (1.2 mg) of NovoSeven® (Novo Nordisk A/S, Denmark) contains 1.2 mg recombinant human Factor VIIa (rhFVIIA), 5.84 mg NaCl, 2.94 mg CaCl2.2H2O, 2.64 mg glycylglycine (GlyGly), 0.14 mg polysorbate 80, and 60.0 mg mannitol; it is reconstituted to pH 5.5 by addition of 2.0 mL water for injection (WFI). When reconstituted, the protein solution is stable for use for 24 hours at room temperature. Thus, no liquid, ready-for-use or concentrated Factor VII products are currently commercially available.

Accordingly, it is highly desirable, and an object of the present invention, to develop agents that inhibit degradation of FVII polypeptides in liquid (particularly aqueous liquid) or solid administration forms. It is particularly desirable to be able to provide an aqueous liquid pharmaceutical composition of a Factor VII polypeptide which provides acceptable control of chemical and/or physical degradation processes such as those outlined above.

International Publication No. WO2005016365 describes liquid, aqueous pharmaceutical compositions comprising a Factor VII polypeptide, a buffering agent, and at least one stabilising agent (iii) comprising a -C(=N-$Z^1$-$R^1$)-NH-$Z^2$-$R^2$ motif, e.g. benzamidine compounds and guanidine compounds such as arginine.

## SUMMARY OF THE INVENTION

The present invention provides compounds of formula (I)

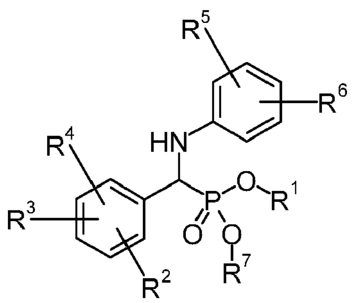

(I)

wherein,

**$R^1$** represents hydrogen, straight or branched ($C_1$-$C_{15}$) alkyl, (cycloalkyl)alkyl, benzyl, 1-arylethyl, 2-arylethyl, heteroarylmethyl, 1-heteroarylethyl, 2-heteroarylethyl, aryl, or heteroaryl, optionally substituted with halogen, amino, hydroxyl, cyano, -$CONH_2$, nitro, acetyl, cyclopropanoyl, or carboxyl;

**$R^2$** represents hydrogen, halogen, methylsulfonylamino, phenylsulfonylamino, or chlorophenylsulfonylamino;

**$R^3$** and **$R^4$** independently represent methoxy, ethoxy, lower alkoxy, benzyloxy, lower alkyl, halogen, amino, hydroxyl, cyano, -$CONH_2$, nitro, acetyl, cyclopropanoyl, carboxylmethyl, or carboxyl;

**$R^5$** represents hydrogen, hydroxyl, or halogen;

**$R^6$** represents -C(=NH)-$NH_2$, -C(=N-OH)-$NH_2$, -C(=$NNH_2$)-$NH_2$, -NH-C(=NH)-$NH_2$, or - C(=NH)-$NHCO_2R^8$, wherein $R^8$ represents lower alkyl, aryl, or arylmethyl, optionally substituted with lower alkyl, alkoxy, or halogen; and

**$R^7$** represents hydrogen, lower alkyl, benzyl, C($R^9R^{10}$)$OR^{11}$, or C(=O)$OR^{12}$, wherein

**$R^9$** and **$R^{10}$** independently represent hydrogen, lower alkyl, or aryl,

**$R^{11}$** and **$R^{12}$** independently represent lower alkyl, aryl, or arylmethyl.

including any and all stereoisomeric form or forms thereof; and any mixture of two or more such compounds of formula I in any ratio; and physiologically tolerable salts and prodrugs thereof.

The present inventors have discovered that compounds of general formula (I) are inhibitors of the proteases involved in the blood coagulation cascade, and may therefore be used as stabilizing additives for formulations of FVII polypeptides, notably aqueous solutions of FVIIa. Blood coagulation Factor VII or analogues thereof ("Factor VII polypeptides"), when formulated as liquid, aqueous pharmaceutical compositions together with at least one stabilising agent with formula (I), exhibit improved stability and thereby allow for prolonged storage before and/or during actual use.

The present invention further provides:

(i) pharmaceutical compositions comprising a FVII polypeptide, such as wild-type FVIIa, rhFVIIa, or analogs or derivatives thereof, and a compound of formula (I);
(ii) methods for preparing a pharmaceutical compositions comprising a FVII polypeptide, such as wild-type FVIIa, rhFVIIa, or analogs thereof, and a compound of formula (I);
(iii) methods for inhibiting a FVII polypeptide, such as wild-type FVIIa, rhFVIIa, or analogs or derivatives thereof;
(iv) the use of compounds of formula (I), in combination with a FVII polypeptide, such as wild-type FVIIa, rhFVIIa, or analogs or derivatives thereof, for the manufacture of pharmaceutical compositions for the treatment of bleeding, haemophilia, or other diseases or symptoms, in which the treatment with a FVII polypeptide is beneficial.

## DETAILED DESCRIPTION OF THE INVENTION

As mentioned above, the present invention provides novel compounds with formula (I) (see above) that inhibit degradation of serine proteases, such as e.g. Factor VII polypeptides in liquid (particularly aqueous liquid) or solid administration forms. Furthermore, the invention provides aqueous liquid pharmaceutical compositions of serine proteases, in particular Factor VII polypeptides, which provide acceptable control of chemical and/or physical degradation processes such as those outlined above. Factor VII or analogues thereof ("Factor VII polypeptides"), when formulated as liquid, aqueous pharmaceutical compositions together with at least one compound with formula (I) exhibits improved stability and thereby allow for prolonged (e.g. 6 months or more) storage before actual use.

Compounds of formula (I) are capable of reversibly inhibiting Factor VIIa and may be employed as stabilizers in formulations or compositions, notable aqueous formulations or compositions, comprising a Factor VII polypeptide (see below). In this connection, compounds of the invention frequently exhibit favourable solubility in water or other aqueous media.

In the present context, the term "alkyl" is intended to indicate a straight or branched saturated monovalent hydrocarbon radical. The term "alkylene" indicates the corresponding diradical. A "lower alkyl" is an alkyl having from 1 to 6 carbon atoms, also denoted as C1-6-alkyl. C1-6-alkyl groups include for instance methyl, ethyl, n-propyl, isopropyl, n-butyl, sec-butyl, isobutyl, tert-butyl, n-pentyl, 2-methylbutyl, 3-methylbutyl, 4-methylpentyl, n-hexyl, 1,1-dimethylpropyl, 1,2-dimethylpropyl, 2,2-dimethylpropyl (neopentyl) and 1,2,2-trimethylpropyl.

In the present context, the term "cycloalkyl" is intended to indicate a cyclic saturated monovalent hydrocarbon radical. A "lower cycloalkyl" is an cycloalkyl having from three to six carbon atoms, also denoted as $C_{1-6}$-cycloalkyl. $C_{1-6}$-cycloalkyl groups include for instance cyclopropyl, cyclobutyl, cyclopentyl, 2-methyl-cyclopentyl, and cyclohexyl.

The term "aryl" as used herein is intended to indicate a mono- or polycyclic carbocyclic aromatic ring radical with for instance 6 to 10 member atoms, or an aromatic ring system radical with for instance from 10 to 22 member atoms. Aryl is also intended to include the partially hydrogenated derivatives of the carbocyclic systems, wherein at least one ring is aromatic. Examples of such partially hydrogenated derivatives include 1,2,3,4-tetrahydronaphthyl, fluorenyl and 1,4-dihydronaphthyl.

The term "heteroaryl" as used herein is intended to indicate a mono- or polycyclic heterocyclic aromatic ring radical with for instance 5 to 13 member atoms, or a heterocyclic aromatic ring system radical with for instance from 13 to 21 member atoms, containing one or more heteroatoms selected from nitrogen, oxygen, and sulfur, wherein N-oxides and sulfur monoxides and sulfur dioxides are permissible heteroaromatic substitutions, such as for instance furyl, thienyl, thiophenyl, pyrrolyl, oxazolyl, thiazolyl, imidazolyl, pyrazolyl isoxazolyl, oxadiazoly, thiadiazolyl, isothiazolyl, 1,2,3-triazolyl, 1,2,4-triazolyl, pyranyl, pyridyl, pyridazinyl, pyrimidinyl, pyrazinyl, 1,2,3-triazinyl, 1,2,4-triazinyl, 1,3,5- triazinyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, tetrazolyl, thiadiazinyl, indolyl, isoindolyl, benzofuryl, benzothienyl, indazolyl, benzimidazolyl, benzthiazolyl, benzisothiazolyl, benzoxazolyl, benzisoxazolyl, purinyl, quinazolinyl, quinolizinyl, quinolinyl, isoquinolinyl, quinoxalinyl, naphthyridinyl, pteridinyl, carbazolyl, azepinyl, diazepinyl, acridinyl and the like. Heteroaryl is also intended to include the partially hydrogenated derivatives of the heterocyclic systems, provided at least one ring comprising a hetero atom is aromatic. Examples of such partially hydrogenated derivatives include 2,3-dihydrobenzofuranyl, pyrrolinyl, pyrazolinyl, indolinyl, oxazolidinyl, oxazolinyl and oxazepinyl.

Examples of "aryl" and "heteroaryl" include, but are not limited to phenyl, biphenylyl, indenyl, fluorenyl, phenanthrenyl, azulenyl, naphthyl (1-naphthyl, 2-naphthyl), anthracenyl (1-anthracenyl, 2-anthracenyl, 3-anthracenyl), thienyl (2-thienyl, 3-thienyl), furyl (2-furyl, 3-furyl), indolyl, oxadiazolyl, isoxazolyl, thiadiazolyl, oxatriazolyl, thiatriazolyl, quinazolin, fluorenyl, xanthenyl, isoindanyl, benzhydryl, acridinyl, thiazolyl, pyrrolyl (1-pyrrolyl, 2-pyrrolyl, 3-pyrrolyl), pyrazolyl (1-pyrazolyl, 3-pyrazolyl, 4-pyrazolyl, 5-pyrazolyl), imidazolyl (1-imidazolyl, 2-imidazolyl, 4-imidazolyl, 5-imidazolyl), triazolyl (1,2,3-triazol-1-yl, 1,2,3-triazol-4-yl 1,2,3-triazol-5-yl, 1,2,4-triazol-3-yl, 1,2,4-triazol-5-yl), oxazolyl (2-oxazolyl, 4-oxazolyl, 5-oxazolyl), isooxazolyl (isooxazo-3-yl, isooxazo-4-yl, isooxaz-5-yl), isothiazolyl (isothiazo-3-yl, isothiazo-4-yl, isothiaz-5-yl) thiazolyl (2-thiazolyl, 4-thiazolyl, 5-thiazolyl), pyridyl (2-pyridyl, 3-pyridyl, 4-pyridyl), pyrimidinyl (2-pyrimidinyl, 4-pyrimidinyl, 5-pyrimidinyl, 6-pyrimidinyl), pyrazinyl, pyridazinyl (3- pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydro-benzo[b]furanyl)), benzo[b]thiophenyl (benzo[b]thiophen-2-yl, benzo[b]thiophen-3-yl, benzo[b]thiophen-4-yl, benzo[b]thiophen-5-yl, benzo[b]thiophen-6-yl, benzo[b]thiophen-7-yl), 2,3-dihydro-benzo[b]thiophenyl (2,3-dihydro-benzo[b]thiophen-2-yl, 2,3-dihydro-benzo[b]thiophen-3-yl, 2,3-dihydro-benzo[b]thiophen-4-yl, 2,3-dihydro-benzo[b]thiophen-5-yl, 2,3-dihydro-benzo[b]thiophen-6-yl, 2,3-dihydro-benzo[b]thiophen-7-yl), indolyl (1-indolyl, 2-indolyl, 3-indolyl, 4-indolyl, 5-indolyl, 6-indolyl, 7-indolyl), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (2-benzoxazolyl, 3-benzoxazolyl, 4-benzoxazolyl, 5-benzoxazolyl, 6-benzoxazolyl, 7-benzoxazolyl), benzothiazolyl (2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepine (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepine (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz[b,f]azepine-4-yl, 10,11-dihydro-5H-dibenz[b,f]azepine-5-yl), benzo[1,3]dioxole (2-benzo[1,3]dioxole, 4-benzo[1,3]dioxole, 5-benzo[1,3]dioxole, 6-benzo[1,3]dioxole, 7-benzo[1,3]dioxole), and tetrazolyl (5-tetrazolyl, N-tetrazolyl).

As used herein, the term "prodrug" includes biohydrolyzable amides and biohydrolyzable esters, acetals, aminals, carbamates, thioaminals, thioacetals, and hydrazones, and also encompasses a) compounds in which the biohydrolyzable functionality in such a prodrug is encompassed in the compound according to the present invention, and b) compounds which may be oxidized or reduced biologically at a given functional group to yield drug substances according to the present invention. Examples of these functional groups include 1,4-dihydropyridine, N-alkylcarbonyl-1,4-dihydropyridine, 1,4-cyclohexadiene, tert-butyl, and the like. The invention also comprises prodrugs of compounds of formula (I).

Stereogenic atoms present in the compounds of formula (I) can independently of each other have R configuration or S configuration. The compounds of formula (I) can be pure enantiomers or mixtures of enantiomers, e.g. racemates, or pure diastereomers or mixtures of diastereomers. The invention also comprises all tautomeric forms of compounds of formula (I).

In the present context, the term "pharmaceutically tolerable salt" is intended to indicate salts which are not harmful to the patient. Such salts include pharmaceutically tolerable acid addition salts, pharmaceutically tolerable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically tolerable inorganic or organic acid addition salts include the pharmaceutically tolerable salts listed in J. Pharm. Sci. 66, 2 (1977), which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. The present invention also comprises pharmaceutically tolerable salts of compounds of formula (I).

As used herein, the term "solvate" is a complex of defined stoichiometry formed by a solute and a solvent. Solvents may be, by way of example, water, ethanol, or acetic acid.

In particular embodiments, the invention relates to compounds of formula (I), wherein $R^1$ represents straight or branched ($C_1$-$C_{15}$) alkyl, (cycloalkyl)alkyl, benzyl, 1-arylethyl, 2-arylethyl, heteroarylmethyl, 1-heteroarylethyl, or 2-heteroarylethyl, optionally substituted with halogen, amino, or hydroxyl.

In a further embodiment, $R^1$ represents methyl, ethyl, propyl, butyl, isopropyl, or benzyl, optionally substituted with halogen, amino, or hydroxyl. In another embodiment, $R^1$ represents methyl or benzyl.

In a particular embodiment, $R^1$ represents methyl, ethyl or benzyl, and $R^7$ represents hydrogen.

In particular embodiments, $R^3$ and $R^4$ independently represent methoxy, ethoxy, lower alkoxy, benzyloxy, lower alkyl, halogen, or carboxyl.

In particular embodiments, $R^2$ represents hydrogen, methylsulfonylamino, phenylsulfonylamino, or halogen, and $R^3$ and $R^4$ independently represent methoxy, ethoxy, lower alkoxy, benzyloxy, lower alkyl, or carboxyl.

In a further embodiment, $R^2$ represents hydrogen or halogen, and $R^3$ and $R^4$ independently represent methoxy, ethoxy, isopropoxy, or benzyloxy.

In yet another embodiment, $R^2$ represents hydrogen, and $R^3$ and $R^4$ represent 3-methoxy and 4-benzyloxy. In another embodiment, $R^2$ represents hydrogen or fluorine, and $R^3$ and $R^4$ represent 3,5-diethoxy.

In particular embodiments, $R^5$ represents hydrogen or hydroxyl.

In a particular embodiment, $R^5$ represents hydrogen or hydroxyl, and $R^6$ represents - C(=NH)-NH2.

In a particular embodiment, $R^5$ represents hydrogen and $R^6$ represents 4-C(=NH)-NH2.

In one embodiment, the compound with formula (I) is selected from the list of:

[(4-benzyloxy-3-methoxyphenyl)(4-carbamimidoylphenylamino)methyl]phosphonic acid dimethyl ester;
[(4-benzyloxy-3-methoxyphenyl)(4-carbamimidoylphenylamino)methyl]phosphonic acid monomethyl ester;
[(4-benzyloxy-3-methoxyphenyl)(4-carbamimidoylphenylamino)methyl]phosphonic acid dibenzyl ester;
[(4-benzyloxy-3-methoxyphenyl)(4-carbamimidoylphenylamino)methyl]phosphonic acid monobenzyl ester;
[(4-benzyloxy-3-ethoxyphenyl)(4-carbamimidoylphenylamino)methyl]phosphonic acid dimethyl ester;
[(4-benzyloxy-3-ethoxyphenyl)(4-carbamimidoylphenylamino)methyl]phosphonic acid monomethyl ester;
[(4-benzyloxy-3-ethoxyphenyl)(4-carbamimidoylphenylamino)methyl]phosphonic acid dibenzyl ester;
[(4-benzyloxy-3-ethoxyphenyl)(4-carbamimidoylphenylamino)methyl]phosphonic acid monobenzyl ester;
[(4-isopropoxy-3-ethoxyphenyl)(4-carbamimidoylphenylamino)methyl]phosphonic acid dimethyl ester;
[(4-isopropoxy-3-ethoxyphenyl)(4-carbamimidoylphenylamino)methyl]phosphonic acid monomethyl ester;
[(4-isopropoxy-3-ethoxyphenyl)(4-carbamimidoylphenylamino)methyl]phosphonic acid dibenzyl ester;
[(4-isopropoxy-3-ethoxyphenyl)(4-carbamimidoylphenylamino)methyl]phosphonic acid monobenzyl ester;
[(4-benzyloxy-3-ethoxy-6-methylsulfonylaminophenyl)(4-carbamimidoylphenylamino)methyl]phosphonic acid dimethyl ester;
[(4-benzyloxy-3-ethoxy-6-methylsulfonylaminophenyl)(4-carbamimidoylphenylamino)methyl]phosphonic acid monomethyl ester;
[(4-benzyloxy-3-ethoxy-6-methylsulfonylaminophenyl)(4-carbamimidoylphenylamino)methyl]phosphonic acid dibenzyl ester;
[(4-benzyloxy-3-ethoxy-6-methylsulfonylaminophenyl)(4-carbamimidoylphenylamino)methyl]phosphonic acid monobenzyl ester; [(3,4-diethoxyphenyl)(4-carbamimidoylphenylamino)methyl]phosphonic acid dimethyl ester;
[(3,4-diethoxyphenyl)(4-carbamimidoylphenylamino)methyl]phosphonic acid monomethyl ester;
[(3,4-diethoxyphenyl)(4-carbamimidoylphenylamino)methyl]phosphonic acid dibenzyl ester;
[(3,4-diethoxyphenyl)(4-carbamimidoylphenylamino)methyl]phosphonic acid monobenzyl ester;
[(3,5-diethoxyphenyl)(4-carbamimidoylphenylamino)methyl]phosphonic acid dimethyl ester;
[(3,5-diethoxyphenyl)(4-carbamimidoylphenylamino)methyl]phosphonic acid monomethyl ester;
[(3,5-diethoxyphenyl)(4-carbamimidoylphenylamino)methyl]phosphonic acid dibenzyl ester;
[(3,5-diethoxyphenyl)(4-carbamimidoylphenylamino)methyl]phosphonic acid monobenzyl ester;
including any and all stereoisomeric form or forms thereof;
and any mixture of two or more such compounds of formula I in any ratio;
and physiologically tolerable salts and prodrugs thereof.

In another embodiment, the compound with formula (I) is selected from the list of:

[(4-benzyloxy-3-methoxyphenyl)(4-carbamimidoylphenylamino)methyl]phosphonic acid dimethyl ester;
[(4-benzyloxy-3-methoxyphenyl)(4-carbamimidoylphenylamino)methyl]phosphonic acid monomethyl ester;
[(4-benzyloxy-3-methoxyphenyl)(4-carbamimidoylphenylamino)methyl]phosphonic acid dibenzyl ester;
[(4-benzyloxy-3-methoxyphenyl)(4-carbamimidoylphenylamino)methyl]phosphonic acid monobenzyl ester;
[(3,5-diethoxyphenyl)(4-carbamimidoylphenylamino)methyl]phosphonic acid dimethyl ester;
[(3,5-diethoxyphenyl)(4-carbamimidoylphenylamino)methyl]phosphonic acid monomethyl ester;
[(3,5-diethoxyphenyl)(4-carbamimidoylphenylamino)methyl]phosphonic acid dibenzyl ester;
[(3,5-diethoxyphenyl)(4-carbamimidoylphenylamino)methyl]phosphonic acid monobenzyl ester;
including any and all stereoisomeric form or forms thereof;
and any mixture of two or more such compounds of formula I in any ratio;
and physiologically tolerable salts and prodrugs thereof.

The compounds of formula I are reversible inhibitors of Factor VII polypeptides (in their activated form). Preferably, they are specific inhibitors of Factor VII polypeptides. As used herein, the term specific when used in reference to the inhibition of Factor VIIa activity means that a compound of the formula I can inhibit Factor VII activity without substantially inhibiting the activity of other specified proteases involved in the blood coagulation and/or the fibrinolysis pathway including, for example, Factor Xa, plasmin, thrombin, Factor IXa, Factor XIa, Factor XIIa and tissue-plasminogen activator (tPA) (using the same concentration of the inhibitor).

The present specification describes assays and methods (see below) for determining the inhibition constant ($K_i$) for a Factor VII polypeptide as well as other specified proteases involved in the blood coagulation and/or the fibrinolysis pathway.

In preferred embodiments of the invention, the compounds of formula I exhibit at least one of the following:

- a $K_i$(activated Factor VII polypeptide) of 1/10 or less (such as 1/20 or less, 1/50 or less, or 1/100 or less) of $K_i$(Factor Xa) (using the same concentration of the inhibitor);
- a $K_i$(activated Factor VII polypeptide) of 1/10 or less (such as 1720 or less, 1/50 or less, or 1/100 or less) of $K_i$ (plasmin) (using the same concentration of the inhibitor);
- a $K_i$(activated Factor VII polypeptide) of 1/10 or less (such as 1/20 or less, 1/50 or less, or 1/100 or less) of $K_i$ (thrombin) (using the same concentration of the inhibitor);
- a $K_i$(activated Factor VII polypeptide) of 1/10 or less (such as 1/20 or less, 1/50 or less, or 1/100 or less) of $K_i$(Factor IXa) (using the same concentration of the inhibitor);
- a $K_i$(activated Factor VII polypeptide) of 1/10 or less (such as 1/20 or less, 1/50 or less, or 1/100 or less) of $K_i$(Factor XIa) (using the same concentration of the inhibitor);
- a $K_i$(activated Factor VII polypeptide) of 1/10 or less (such as 1/20 or less, 1/50 or less, or 1/100 or less) of $K_i$(Factor XIIa) (using the same concentration of the inhibitor);
- a $K_i$(activated Factor VII polypeptide) of 1/10 or less (such as 1/20 or less, 1/50 or less, or 1/100 or less) of $K_i$(tPA) (using the same concentration of the inhibitor).

*Factor VII polypeptides*

As used herein, the terms "Factor VII polypeptide " or "FVII polypeptide" means any protein comprising the amino acid sequence 1-406 of wild-type human Factor VIIa (i.e., a polypeptide having the amino acid sequence disclosed in U.S. Patent No. 4,784,950), variants thereof as well as Factor VII-related polypeptides, Factor VII derivatives and Factor VII conjugates. This includes FVII variants, Factor VII-related polypeptides, Factor VII derivatives and Factor VII conjugates exhibiting substantially the same or improved biological activity relative to wild-type human Factor VIIa.

The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like.

"Factor VII" or "Factor VIIa" within the above definition also includes natural allelic variations that may exist and occur from one individual to another. Also, degree and location of glycosylation or other post-translation modifications may vary depending on the chosen host cells and the nature of the host cellular environment.

As used herein, "wild type human FVIIa" is a polypeptide having the amino acid sequence disclosed in U.S. Patent No. 4,784,950.

As used herein, "Factor VII-related polypeptides" encompasses polypeptides, including variants, in which the Factor VIIa biological activity has been substantially modified, such as reduced, relative to the activity of wild-type Factor VIIa. These polypeptides include, without limitation, Factor VII or Factor VIIa into which specific amino acid sequence alterations have been introduced that modify or disrupt the bioactivity of the polypeptide.

The term "Factor VII derivative" as used herein, is intended to designate a FVII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, acylation, ester formation or amide formation or the like. This includes but is not limited to PEGylated human Factor VIIa, cysteine-PEGylated human Factor VIIa and variants thereof. Non-limiting examples of Factor VII derivatives includes GlycoPegylated FVII derivatives as disclosed in WO 03/31464 and US Patent applications US 20040043446, US 20040063911, US 20040142856, US 20040137557, US 20040132640, and WO 2007/022512 (Neose Technologies, Inc.); FVII conjugates as disclosed in WO 01/04287, US patent application 20030165996, WO 01/58935, WO 03/93465 (Maxygen ApS) and WO 02/02764, US patent application 20030211094 (University of Minnesota). PEGylated human Factor VIIa encompasses any Factor VIIa to which one or more PEG moieties has been attached. The PEG molecule may be attached to any part of the Factor VIIa polypeptide, including any amino acid residue or

carbohydrate moiety of the Factor VIIa polypeptide. The term "cysteine-PEGylated human Factor VIIa" refers to Factor VIIa having a PEG molecule conjugated to a sulfhydryl group of a cysteine introduced in human Factor VIIa to form a Factor VIIa sequence variant.

The PEG may be any branched or unbranched poly(ethylenglycol) having any chain length, including, without limitation, branched PEGs having a molecular weight of from about 5KDa to about 80 KDa, such as, e.g., about 1 KDa, 2 KDa, 5 KDa, 10 KDa, 20 KDa, 30 KDa, and 40 KDa).

The term "improved biological activity" refers to FVII polypeptides with i) substantially the same or increased proteolytic activity compared to recombinant wild type human Factor VIIa or ii) to FVII polypeptides with substantially the same or increased TF binding activity compared to recombinant wild type human Factor VIIa or iii) to FVII polypeptides with substantially the same or increased half life in blood plasma compared to recombinant wild type human Factor VIIa.

Non-limiting examples of Factor VII variants having substantially the same or increased proteolytic activity compared to recombinant wild type human Factor VIIa include S52A-FVIIa, S60A-FVIIa ( Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189 (corresponding to WO 02/077218); and FVII variants exhibiting increased proteolytic stability as disclosed in WO 02/38162 (Scripps Research Institute); FVII variants having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and FVII variants as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of FVII variants having increased biological activity compared to wild-type FVIIa include FVII variants as disclosed in WO 01/83725, WO 02/22776, WO 02/077218, WO 03/027147, WO 04/029090, WO 05/075635, and European patent application with application number 05108713.8 (Novo Nordisk A/S), WO 02/38162 (Scripps Research Institute); and FVIIa variants with enhanced activity as disclosed in JP 2001061479 (Chemo-Sero-Therapeutic Res Inst.).

Examples of variants of factor VII include, without limitation, P10Q-FVII, K32E-FVII, P10Q/K32E-FVII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V158T/E296V/M298Q-FVII, S314E/L305V/V158T/K337A/M298Q-FVII, S314E/L305V/V158T/E296V/K337A-FVII, S314E/L305V/V158D/K337A/M298Q-FVII, S314E/L305V/ V158D/E296V/K337A-FVII, S314E/L305V/V158D/E296V/M298Q/K337A-FVII, S314E/L305V/V158T/E296V/ M298Q/K337A-FVII, K316H/L305V/K337A-FVII, K316H/L305V/V158D-FVII, K316H/L305V/E296V-FVII, K316H/ L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/ L305V/V158D/E296V-FVII, K316H/L305V/V158T/M298Q-FVII, K316H/L305V/V158T/E296V-FVII, K316H/ L305V/E296V/M298Q-FVII, K316H/L305V/V158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/ K337A/M298Q-FVII, K316H/L305V/V158D/E296V/K337A -FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/ L305V/E296V-FVII, K316Q/L305V/M298Q-FVII, K316Q/L305V/V158T-FVII, K316Q/L305V/K337A/V158T-FVII, K316Q/ L305V/K337A/M298Q-FVII, K316Q/L305V/K337A/E296V-FVII, K316Q/L305V/K337A/V158D-FVII, K316Q/L305V/ V158D/M298Q-FVII, K316Q/L305V/V158D/E296V-FVII, K316Q/L305V/V158T/M298Q-FVII, K316Q/L305V/ V158T/E296V-FVII, K316Q/L305V/E296V/M298Q-FVII, K316Q/L305V/V158D/E296V/M298Q-FVII, K316Q/L305V/ V158T/E296V/M298Q-FVII, K316Q/L305V/V158T/K337A/M298Q-FVII, K316Q/L305V/V158T/E296V/K337A-FVII, K316Q/L305V/ V158D/K337A/M298Q-FVII, K316Q/L305V/V158D/E296V/K337A -FVII, K316Q/L305V/ V158D/E296V/M298Q/K337A-FVII, K316Q/L305V/V158T/E296V/M298Q/K337A-FVII, F374Y/K337A-FVII, F374Y/ V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/ L305V/ V158D-FVII, F374Y/L305V/E296V-FVII, F374Y/L305V/M298Q-FVII, F374Y/ L305V/V158T-FVII, F374Y/ L305V/S314E-FVII, F374Y/K337A/S314E-FVII, F374Y/K337A/V158T-FVII, F374Y/ K337A/M298Q-FVII, F374Y/ K337A/E296V-FVII, F374Y/K337A/V158D-FVII, F374Y/V158D/S314E-FVII, F374Y/ V158D/M298Q-FVII, F374Y/V158D/E296V-FVII, F374Y/V158T/S314E-FVII, F374Y/V158T/M298Q-FVII, F374Y/ V158T/E296V-FVII, F374Y/E296V/S314E-FVII, F374Y/S314E/M298Q-FVII, F374Y/E296V/M298Q-FVII, F374Y/ L305V/K337A/V158D-FVII, F374Y/L305V/K337A/E296V-FVII, F374Y/L305V/K337A/M298Q-FVII, F374Y/ L305V/K337A/V158T-FVII, F374Y/ L305V/K337A/S314E-FVII, F374Y/L305V/V158D/E296V-FVII, F374Y/ L305V/V158D/M298Q-FVII, F374Y/ L305V/V158D/S314E-FVII, F374Y/L305V/E296V/M298Q-FVII, F374Y/ L305V/ E296V/V158T-FVII, F374Y/ L305V/E296V/S314E-FVII, F374Y/L305V/M298Q/V158T-FVII, F374Y/L305V/ M298Q/S314E-FVII, F374Y/ L305V/ V158T/S314E-FVII, F374Y/K337A/S314E/V158T-FVII, F374Y/K337A/ S314E/M298Q-FVII, F374Y/ K337A/S314E/E296V-FVII, F374Y/K337A/S314E/V158D-FVII, F374Y/K337A/V158T/ M298Q-FVII, F374Y/ K337A/V158T/E296V-FVII, F374Y/K337A/M298Q/E296V-FVII, F374Y/K337A/M298Q/V158D-FVII, F374Y/K337A/ E296V/V158D-FVII, F374Y/V158D/S314E/M298Q-FVII, F374Y/V158D/S314E/E296V-FVII, F374Y/ V158D/M298Q/ E296V-FVII, F374Y/V158T/S314E/E296V-FVII, F374Y/V158T/S314E/M298Q-FVII, F374Y/V158T/ M298Q/E296V-FVII, F374Y/E296V/S314E/M298Q-FVII, F374Y/L305V/M298Q/K337A/S314E-FVII, F374Y/L305V/ E296V/K337A/ S314E-FVII, F374Y/E296V/M298Q/K337A/S314E-FVII, F374Y/L305V/E296V/M298Q/K337A -FVII, F374Y/L305V/E296V/M298Q/S314E-FVII, F374Y/V158D/E296V/M298Q/K337A-FVII, F374Y/V158D/E296V/M298Q/ S314E-FVII, F374Y/L305V/V158D/K337A/S314E-FVII, F374Y/V158D/M298Q/K337A/S314E-FVII, F374Y/V158D/ E296V/K337A/S314E-FVII, F374Y/L305V/V158D/E296V/M298Q-FVII, F374Y/L305V/V158D/M298Q/K337A-FVII, F374Y/L305V/V158D/E296V/K337A-FVII, F374Y/L305V/V158D/M298Q/S314E-FVII, F374Y/L305V/V158D/E296V/ S314E-FVII, F374Y/V158T/E296V/M298Q/K337A-FVII, F374Y/V158T/E296V/M298Q/S314E-FVII, F374Y/L305V/ V158T/K337A/S314E-FVII, F374Y/V158T/M298Q/K337A/S314E-FVII, F374Y/V158T/E296V/K337A/S314E-FVII, F374Y/L305V/V158T/E296V/M298Q-FVII, F374Y/L305V/V158T/M298Q/K337A-FVII, F374Y/L305V/V158T/E296V/ K337A-FVII, F374Y/L305V/V158T/M298Q/S314E-FVII, F374Y/L305V/V158T/E296V/S314E-FVII, F374Y/E296V/ M298Q/K337A/V158T/S314E-FVII, F374Y/V158D/E296V/M298Q/K337A/S314E-FVII, F374Y/L305V/V158D/ E296V/ M298Q/S314E-FVII, F374Y/L305V/E296V/M298Q/V158T/S314E-FVII, F374Y/L305V/E296V/M298Q/ K337A/V158T-FVII, F374Y/L305V/E296V/K337A/V158T/S314E-FVII, F374Y/L305V/M298Q/K337A/V158T/S314E-FVII, F374Y/ L305V/V158D/E296V/M298Q/K337A-FVII, F374Y/L305V/V158D/E296V/K337A/S314E-FVII, F374Y/ L305V/ V158D/M298Q/K337A/S314E-FVII, F374Y/L305V/E296V/M298Q/K337A/V158T/S314E-FVII, F374Y/L305V/ V158D/ E296V/M298Q/K337A/S314E-FVII, S52A-Factor VII, S60A-Factor VII; R152E-Factor VII, S344A-Factor VII, T106N-FVII, K143N/N145T-FVII, V253N-FVII, R290N/A292T-FVII, G291N-FVII, R315N/V317T-FVII, K143N/ N145T/R315N/V317T-FVII; and FVII having substitutions, additions or deletions in the amino acid sequence from 233Thr to 240Asn; FVII having substitutions, additions or deletions in the amino acid sequence from 304Arg to 329Cys; and FVII having substitutions, additions or deletions in the amino acid sequence from 153Ile to 223Arg.

Thus, substitution variants in a factor VII polypeptide include, without limitation substitutions in positions P10, K32, L305, M306, D309, L305, L305, F374, V158, M298, V158, E296, K337, M298, M298, S336, S314, K316, K316, F374, S52, S60, R152, S344, T106, K143, N145, V253, R290, A292, G291, R315, V317, and substitutions, additions or deletions in the amino acid sequence from T233 to N240 or from R304 to C329; or from I153 to R223, or combinations thereof, in particular variants such as P10Q, K32E, L305V, M306D, D309S, L305I, L305T, F374P, V158T, M298Q, V158D, E296V, K337A, M298Q, M298K, S336G, S314E, K316H, K316Q, F374Y, S52A, S60A, R152E, S344A, T106N, K143N, N145T, V253N, R290N, A292T, G291N, R315N, V317T, and substitutions, additions or deletions in the amino acid sequence from T233 to N240, or from R304 to C329, or from I153 to R223, or combinations thereof.

The biological activity of Factor VII (as Factor VIIa) in blood clotting derives from its ability to (i) bind to tissue factor (TF) and (ii) catalyze the proteolytic cleavage of Factor IX or Factor X to produce activated Factor IX or X (Factor IXa or Xa, respectively). Human Factor VIIa biological activity may be quantified by an assay measuring the ability of a preparation to promote blood clotting using Factor VII-deficient plasma and thromboplastin, as described, e.g., in U.S. Patent No. 5,997,864. In this assay, biological activity is expressed as the reduction in clotting time relative to a control sample and is converted to "Factor VII units" by comparison with a pooled human serum standard containing 1 unit/ml Factor VII activity. Alternatively, Factor VII polypeptides may also be assayed for specific activities ("clot activity") by using a one-stage coagulation assay. For this purpose, the sample to be tested is diluted in 50 mM PIPES-buffer (pH

7.5), 0.1% BSA and 40 μl is incubated with 40 μl of Factor VII deficient plasma and 80 μl of human recombinant tissue factor containing 10 mM Ca2+ and synthetic phospholipids. Coagulation times (clotting times) are measured and compared to a standard curve using a reference standard in a parallel line assay. Alternatively, Factor VIIa biological activity may be quantified by (i) measuring the ability of Factor VIIa to produce Factor Xa in a system comprising TF embedded in a lipid membrane and Factor X. (Persson et al., J. Biol. Chem. 272:19919-19924, 1997); (ii) measuring Factor X hydrolysis in an aqueous system; (iii) measuring its physical binding to TF using an instrument based on surface plasmon resonance (Persson, FEBS Letts. 413:359-363, 1997) and (iv) measuring hydrolysis of a synthetic substrate.

Factor VII variants having, in the activated (FVIIa) form, substantially the same or improved biological activity relative to wild-type Factor VIIa encompass those that exhibit at least about 10%, preferably at least about 25%, more preferably at least about 50%, even more preferably at least about 75% and most preferably at least about 90% of the specific activity of Factor VIIa that has been produced in the same cell type, when tested in one or more of a clotting assay, proteolysis assay or TF binding assay as described above.

*Processes for preparation* of *compounds of the invention*

Compounds of formula (I) according to the invention may generally be prepared by procedures A and B as described in the following:

*Method (A)*

The compounds of formula (I) according to this invention in which $R^7$ is different from hydrogen, may be prepared as follows:

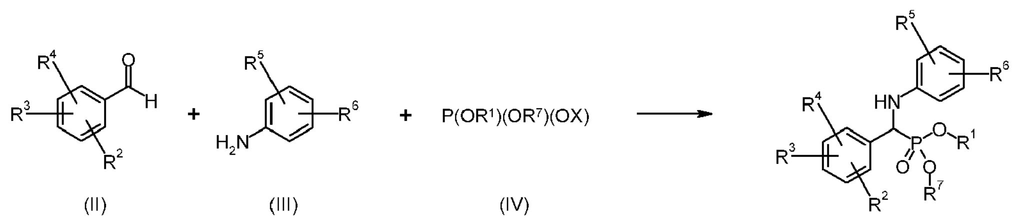


(II) (III) (IV) (I)

A mixture of an aldehyde of formula (II), an aniline of formula (III), optionally as salt of a strong acid (e.g. as hydrochloride), and a phosphite of formula (IV),
wherein X represents hydrogen, lower alkyl, straight or branched ($C_1$-$C_{15}$) alkyl, (cycloalkyl)alkyl, benzyl, 1-arylethyl, 2-arylethyl, heteroarylmethyl, 1-heteroarylethyl, 2-heteroarylethyl, aryl, or heteroaryl, optionally substituted with halogen, amino, hydroxyl, cyano, -$CONH_2$, nitro, acetyl, cyclopropanoyl, carboxyl, benzyl, $C(R^9R^{10})OR^{11}$, or $C(=O)OR^{12}$,

in an appropriate solvent such as acetic acid, THF, dioxane, water, acetonitrile, DMF, NMP, or mixtures thereof, is stirred at room temperature or at higher temperatures, until one of the three starting materials is consumed. The product (I) is isolated and purified using conventional techniques, such as crystallization or chromatography.

*Method (B)*

Compounds of general formula (I), in which $R^7$ represents hydrogen, may be prepared as follows:

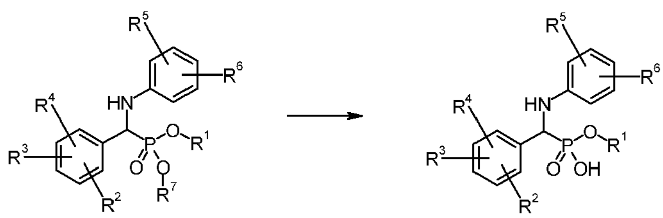

(I) (I')

To the product of *method (a)* (above) with formula (I), in which $R^7$ is not hydrogen, an aqueous solution of sodium hydroxide or an equivalent reagent (e.g. KOH, LiOH, CsOH) is added, followed by the optional addition of an

organic solvent such as THF or dioxane. The resulting mixture is stirred at room temperature or at higher temperatures until the starting material is consumed. The product (I') is isolated and purified using conventional techniques, such as crystallization or chromatography.

*Pharmaceutical formulation and administration of Factor VII polypeptides*

In general, an aqueous, liquid Factor VII polypeptide formulation or composition of the invention (aqueous pharmaceutical composition of the invention) will - irrespective of whether the aqueous formulation is present in aqueous liquid form from the start, or is produced by dissolution/reconstitution of a substantially solid formulation (e.g. a lyophilized preparation) by addition of water or another aqueous carrier or vehicle - in general, suitably be administered parenterally, i.e., intravenously, subcutaneously, or intramuscularly, or by continuous or pulsatile infusion.

For use in human subjects, Factor VII polypeptide compositions of the invention for parenteral administration will, in addition to a compound of formula I or a physiologically tolerable salt thereof in an appropriate concentration, normally comprise the Factor VII polypeptide in combination with, preferably dissolved in, a pharmaceutically acceptable aqueous carrier. A variety of aqueous carriers may be used, such as water, buffered water, 0.4% saline, 0.3% glycine and the like. Factor VII polypeptides in the context of the invention may also be formulated into liposome preparations for delivery or targeting to the sites of injury. Liposome preparations are generally described in, e.g., US 4,837,028, US 4,501,728 and US 4,975,282. The compositions may be sterilised by conventional, well-known sterilisation techniques. The resulting aqueous solutions may be packaged for use as such, or they may be filtered under aseptic conditions and lyophilized, the lyophilized preparation being combined with sterile water or a sterile aqueous solution (carrier, vehicle) prior to administration. The compositions may further contain pharmaceutically acceptable auxiliary substances as required to approximate physiological conditions and/or to enhance the chemical and/or physical stability of the the composition. These include:

*pH-adjusting and/or buffering agents*, e.g. citrate (sodium or potassium), acetate (ammonium, sodium or calcium), histidine (L-histidine), malate, phosphate (sodium or potassium), tartaric acid, succinic acid, MES (2-N-morpholinoethanesulfonic acid), HEPES (4-(2-hydroxy-ethyl)-piperazine-1-ethane-sulfonic acid), imidazole, TRIS [tris(hydroxymethyl)aminomethane], lactate, and glutamate. The buffer concentration range is chosen to maintain the preferred pH of the solution. The buffering agent may also be a mixture of two or more buffering agents, e.g. a mixture of two such agents, such that the mixture is able to provide a pH value in the specified range. In one embodiment, the buffer is a mixture of citrate and at least one of the buffers acetate (ammonium, sodium or calcium), histidine (L-histidine), malate, phosphate (sodium or potassium), tartaric acid, succinic acid, MES, HEPES, imidazole, TRIS, lactate and glutamate. The total concentration of buffer agent(s) is typically in the range of from about 1 mM to about 100 mM, such as from about 1 mM to about 50 mM, often from about 1 mM to about 25 mM, e.g. from about 2 mM to about 20 mM.

*calcium salts:* the compositions - whether initially in liquid, freeze-dried or reconstituted form - may optionally contain a calcium salt. The calcium salt may be present in a low concentration, such as, e.g., from about 0.1 mM to about 5 mM; it may be present in a medium concentration, such as, e.g., from about 5 mM to about 15 mM; or it may be present in a higher concentration, such as, e.g., from about 15 mM to about 1000 mM. In one aspect, the calcium salt is selected from: calcium chloride, calcium acetate, calcium gluconate and calcium laevulate, and mixtures of two or more thereof. Alternatively, the concentration of calcium ions in the composition may be below 0.1 mM.

*tonicity-adjusting agents* (tonicity-modifying substances which contribute to the osmolality of the the formulation), e.g. amino acids, small peptides (having, e.g., from 2 to 5 amino acid residues), neutral salts, mono- or disaccharides, polysaccharides, sugar alcohols, or mixtures of at least two of such substances. Specific examples include, but are not limited to, sodium chloride, potassium chloride, sodium citrate, sucrose, glucose and mannitol. The concentration of tonicity-adjusting agent is adjusted to near isotonicity, depending on the other ingredients present in the formulation. In general, tonicity-adjusting agents are incorporated in a concentration of from about 1 to about 500 mM, such as from about 1 to about 300 mM, often from about 10 to about 200 mM, e.g. from about 20 to about 150 mM, depending on the other ingredients present. Neutral salts such as, e.g., sodium chloride or potassium chloride may be used. The term "neutral salt" indicates a salt that is substantially neither acidic nor basic, i.e. has little or no effect on formulation pH when dissolved;

*surfactants,* typically a non-ionic surfactant, suitably of the polysorbate or Tween™ type (e.g. Polysorbate™ 20 or 80, or Tween™ 80), or of the poloxamer or Pluronic™ type (e.g. Poloxamer™ 188 or 407). The amount of surfactant incorporated may typically range from about 0.005 to about 1% weight/weight (w/w), with amounts of from about 0.005 to about 0.1% w/w, such as from about 0.005 to 0.02% w/w, typically being preferred. In some situations,

relatively high concentrations, e.g. up to about 0.5% w/w, are desirable to maintain protein stability. However, the levels of surfactant used in actual practice are customarily limited by clinical practice;

antioxidants, e.g. ascorbic acid, cysteine, homocysteine, cystine, cysstathionine, methionine, glutathione, or peptides containing cysteine or methionine; methionine, in particular L-methionine, is typically a very suitable antioxidant. An antioxidant is typically incorporated in a concentration of from about 0.1 to about 2 mg/ml;

preservatives (included in the formulation to retard microbial growth, thereby permitting, for example, "multiple use" packaging of the FVII polypeptide). Examples of preservatives include phenol, benzyl alcohol, ortho-cresol, meta-cresol, para-cresol, methylparaben, propylparaben, benzalconium chloride, and benzethonium chloride. A preservative is typically incorporated in a concentration of from about 0.1 to about 2 mg/ml, depending on the pH range and type of preservative.

The concentration of Factor VII polypeptide in the compositions can vary widely, typically from about 0.01% w/w to about 2% w/w (i.e. from about 0.1 mg/ml to about 20 mg/ml), such as from about 0.05% w/w to about 1.5% w/w (i.e. from about 0.5 mg/l to about 15 mg/ml), e.g. from about 0.05% w/w to about 1% w/w (i.e. from about 0.5 mg/ml to about 10 mg/ml), and will be selected primarily on the basis of fluid volumes, viscosities, etc., in accordance with the particular mode of administration selected. In the case of Factor VIIa, concentration is frequently expressed as mg/ml or as International units/ml (IU/ml). 1 mg of FVIIa usually corresponds to 43000-56000 IU or more.

The concentration of a compound (or compounds) of formula I of the invention in a liquid, aqueous pharmaceutical composition of the invention will typically be at least 1 μM. The desirable (or necessary) concentration typically depends on the selected compound (or compounds), more specifically on the binding affinity of the selected compound (s) to the Factor VII polypeptide. In various embodiments, the compound of formula I may be present in a concentration of at least 5 μM, at least 10 μM, at least 20 μM, at least 50 μM, at least 100 μM, at least 150 μM, at least 250 μM, at least 500 μM, at least 1 mM, at least 2 mM, at least 4 mM, at least 5 mM, at least 8 mM, at least 9 mM, at least 10 mM, at least 15 mM, or at least 20 mM, such as, e.g., in the range of 1-10000 μM, 10-10000 μM, 20-10000 μM, 50-10000 μM, 10-5000 μM, 10-2000 μM, 20-5000 μM, 20-2000 μM, 50-5000 μM, 0.1-100 mM, 0.1-75 mM, 0.1-50 mM, 0.1-10 mM, 0.2-75 mM, 0.2-50 mM, 0.2-20 mM, 0.5-75 mM or 0.5-50 mM.

In various embodiments, the molar ratio between the compound of formula I and FVII polypeptide may be: above 0.1, above 0.5, above 1, above 2, above 5, above 10, above 25, above 100, above 250, above 1000, above 2500, or above 5000, such as, e.g., in the range of 0.1-10000, 0.1-5000, 0.1-2500, 0.1-1000, 0.1-250, 0.1-100, 0.1-25, 0.1-10, 0.5-10000, 0.5-5000, 0.5-2500, 0.5-1000, 0.5-250, 0.5-100, 0.5-25, 0.5-10, 1-10000, 1-5000, 1-2500, 1-1000, 1-250, 1-100; 1-25; 1-10, 10-10000, 10-5000, 10-250, 1000-10000, or 1000-5000.

Methods for preparing parenterally administrable compositions will be known or apparent to those skilled in the art, and are described in more detail in, for example, Remington's Pharmaceutical Sciences, 18th edition, Mack Publishing Company, Easton, PA (1990).

For treatment in connection with deliberate interventions (e.g. surgical procedures), Factor VII polypeptides will typically be administered within about 24 hours prior to performing the intervention, and for as much as 7 days or more thereafter.

Administration as a coagulant can be by a variety of routes as described herein. The dose of Factor VII polypeptide (e.g. rhFVIIa) will normally range from about 0.05 mg/day to 500 mg/day, preferably from about 1 mg/day to about 200 mg/day, and more preferably from about 10 mg/day to about 175 mg/day for a 70 kg subject as loading and maintenance doses, depending on the weight of the subject and the severity of the condition.

Compositions containing Factor VII polypeptides may be administered for prophylactic and/or therapeutic treatments. In therapeutic applications, compositions are administered to a subject already suffering from a condition, as described above, in an amount sufficient to cure, alleviate or partially arrest the condition and its complications. An amount adequate to accomplish this is defined as a "therapeutically effective amount". As will be understood by the person skilled in the art, amounts effective for this purpose will depend on the severity of the condition or injury, as well as on the body weight and general physical condition of the subject.

It should be borne in mind that pharmaceutical compositions of FVII polypeptides (e.g. rhFVIIa) are generally employed in connection with life-threatening or potentially life-threatening medical conditions or states, and in such circumstances - in view of the general advantages associated with minimizing quantities of extraneous substances, and taking into account the general lack of immunogenicity of human Factor VII polypeptides - it is possible and may be felt desirable by the treating physician to administer a substantial excess of the Factor VII polypeptide in question.

In prophylactic applications, compositions containing a Factor VII polypeptide are administered to a subject susceptible to, or otherwise at risk of, a disease state or injury in order to enhance the subject's own coagulative capability. The dosage employed for such purposes (which may be termed a "prophylactically effective dose") will once again depend on the subject's body weight and general state of health, but will once again generally range from about 0.05

mg/day to about 500 mg/day, more commonly from about 1.0 mg/day to about 200 mg/day for a 70-kilogram subject.

In the case, specifically, of administration of rhFVIIa to human subjects, dosage levels have generally been in the range of about 90-120 μg/kg body weight per dose. However, there is a current preference for somewhat higher doses, e.g. doses in excess of 150 μg/kg body weight, and in some cases doses of about 250-300 μg/kg.

Single or multiple administration of the composition in question may be carried out using dose levels and dosing regimens selected by the treating physician. For out-patients requiring daily maintenance levels, a Factor VII polypeptide may be administered by continuous infusion, e.g. using a portable pump system.

Local administration of a Factor VII polypeptide, e.g. topical application, may be carried out, e.g., by spraying, by perfusion, by use of a double balloon catheter or a stent, by incorporation into vascular grafts or stents, in the form of hydrogels to coat balloon catheters, or by other well established methods. In any event, the pharmaceutical composition in question should provide a quantity of Factor VII polypeptide which is adequate to effectively treat the subject.

The liquid pharmaceutical preparation should typically be stable for at least six months, and preferably up to 36 months, when stored at temperatures ranging from 2°C to 8°C. It should be understood that the liquid pharmaceutical preparation preferably is stable even at higher temperatures, such as ambient temperature, e.g. 20°C to 30°C, although this often requires a higher content of the inhibitor.

The term "stable" is intended to denote that after storage for 6 months at 2°C to 8°C the initial liquid pharmaceutical preparation retains at least 50% of its initial biological activity. Preferably, the liquid pharmaceutical preparation retains at least 70%, such as at least 80%, or at least 85%, or at least 90%, or at least 95%, of its initial activity after storage for 6 months at 2 to 8°C.

With respect to Factor VII polypeptides, the term "stable" is more particularly intended to denote that (i) after storage for 6 months at 2°C to 8°C the initial liquid pharmaceutical preparation retains at least 50% of its initial biological activity as measured by a one-stage clot assay (Assay 4), or (ii) after storage for 6 months at 2°C to 8°C, the content of heavy chain degradation products (Assay 5) is at the most 40% (w/w) assuming that the initial liquid pharmaceutical preparation comprises no heavy chain degradation products (i.e. only the Factor VII polypeptide is entered into the calculation of the percentage). Preferably, the initial liquid pharmaceutical preparation retains at least 70%, such as at least 80%, or at least 85%, or at least 90%, or at least 95%, of its initial activity after storage for 6 months at 2 to 8°C. Also preferably, the content of heavy chain degradation products in the initial liquid pharmaceutical preparation is at the most 30% (w/w), at the most 25% (w/w), at the most 20% (w/w), at the most 15% (w/w), at the most 10% (w/w), at the most 5% (w/w), or at the most 3% (w/w).

Hence, as outlined above, a "stabilized composition" refers to a composition with increased physical stability, increased chemical stability or increased physical and chemical stability. In general, a composition must be stable during use and storage (in compliance with recommended use and storage conditions) until the expiration date is reached.

In one embodiment the pharmaceutical composition comprising the FVII polypeptide and a compound of formula (I) is stable for more than 6 months of storage (at 2-8°C) and for more than 1 week of usage (at ambient temperature). In a further embodiment the pharmaceutical composition comprising the FVII polypeptide and a compound of formula (I) is stable for more than 24 months of storage (at 2-8°C) and for more than 4 weeks of usage (at ambient temperature). In another, further embodiment the pharmaceutical composition comprising the FVII polypeptide and a compound of formula (I) is stable for more than 36 months of storage (at 2-8°C) and for more than 6 weeks of usage (at ambient temperature).

As will be understood, the liquid, aqueous pharmaceutical compositions defined herein can be used in the field of medicine. Thus, the present invention in particular provides the liquid, aqueous pharmaceutical compositions defined herein for use as a medicament, more particular for use as a medicament for treating condition or disorder against which said Factor VII polypeptide is effective.

Consequently, the present invention also provides the use of the liquid, aqueous pharmaceutical composition as defined herein for the preparation of a medicament for treating a condition or disorder against which said Factor VII polypeptide is effective, as well as a method for treating a condition or disorder against which said Factor VII polypeptide is effective, the method comprising administering to a subject in need thereof an effective amount of the liquid, aqueous pharmaceutical composition as defined herein.

The preparations of the present invention may be used to treat any condition or disorder against which said Factor VII polypeptide is effective, such as, without limitation, bleeding disorders, including those caused by clotting factor deficiencies (e.g., congenital haemophilia A with and without inhibitors, acquired haemophilia A, congenital haemophilia B with and without inhibitors, acquired haemophilia B, coagulation Factor XI deficiency, coagulation Factor VII deficiency), von Willebrand's disease, platelet disorders or deficiencies (e.g., low platelet count), or thrombocytopenia. As used herein the term "bleeding disorder" reflects any defect, congenital, acquired or induced, of cellular or molecular origin that is manifested in bleedings.

Conditions or disorders against which said Factor VII polypeptide is effective also include bleedings in subjects who experience extensive tissue damage, e.g. in association with surgery or trauma including, without limitation, bleedings associated with spinal or cardiac surgery, orthopedic surgery (e.g. hips, elbows, knees), or laparoscopic surgery, penetrating or blunt trauma, head trauma including traumatic brain injury, intracerebral haemorrhage, bleedings associated with induced defective haemostasis, such as bleedings associated with anticoagulant therapy or antifibrinolytic therapy, and uncontrolled and excessive bleeding from any cause. In case of extensive tissue damage, the normal haemostatic mechanism may be overwhelmed by the demand of immediate haemostasis and bleedings may develop in spite of an otherwise normal haemostatic mechanism.
Included are also bleedings in organs with limited possibility for surgical haemostasis such as e.g. the brain, inner ear region, eyes, liver, lung, tumour tissue, and gastrointestinal tract as well as when bleeding is diffuse (haemorrhagic gastritis and profuse uterine bleeding). Common for all these situations is the difficulty to provide haemostasis by surgical techniques (sutures, clips, etc.).

The term "effective amount" is the effective dose to be determined by a qualified practitioner, who may titrate dosages to achieve the desired response. Factors for consideration of dose will include potency, bioavailability, desired pharmacokinetic/pharmacodynamic profiles, condition of treatment, patient-related factors (e.g. weight, health, age, etc.), presence of co-administered medications (e.g., anticoagulants), time of administration, or other factors known to a medical practitioner.

The term "treatment" is defined as the management and care of a subject, e.g. a mammal, in particular a human, for the purpose of combating the disease, condition, or disorder and includes the administration of a Factor VII polypeptide to prevent the onset of the symptoms or complications, or alleviating the symptoms or complications, or eliminating the disease, condition, or disorder. Pharmaceutical compositions according to the present invention containing a Factor VII polypeptide may be administered parenterally to subjects in need of such a treatment. Parenteral administration may be performed by subcutaneous, intramuscular or intravenous injection by means of a syringe, optionally a pen-like syringe. Alternatively, parenteral administration can be performed by means of an infusion pump.

In important embodiments, the pharmaceutical composition is adapted to subcutaneous, intramuscular or intravenous injection according to methods known in the art.

The pharmaceutical composition comprising a Factor VII polypeptide and one or more compounds (I) according to the present invention may further contain, or be used in conjunction with, one or more coagulation factors, such as, without limitation, Factor XIII, Factor VIII, Factor IX, another Factor VII polypeptide, or FEIBA™.

*Further aspects of the invention*

As already indicated to some extent above, further aspects of the present invention include the following:

- a pharmaceutical composition (e.g. a liquid, aqueous pharmaceutical composition) comprising: one or more compounds, or physiologically tolerable salts thereof, according to the invention; and a Factor VII polypeptide (e.g. wild-type human FVIIa, such as rhFVIIa);

- a method of preparing a composition comprising a Factor VII polypeptide (e.g. wild-type human FVIIa, such as rhFVIIa), comprising: adding a compound, or a physiologically tolerable salt thereof, according to the invention to a sample containing the Factor VII polypeptide; or adding the Factor VII polypeptide to a sample containing a compound, or a physiologically tolerable salt thereof, according to the invention; in a method of this type, the compound or salt thereof and/or the Factor VII polypeptide may be present in a liquid, aqueous medium.

- a pharmaceutical composition prepared by a method according to the invention;

- a method of inhibiting a Factor VII polypeptide (e.g. wild-type FVIIa, such as rhFVIIa), comprising: adding a compound, or a physiologically tolerable salt thereof, according to the invention to a sample containing the Factor VII polypeptide; or adding the Factor VII polypeptide to a sample containing a compound, or a physiologically tolerable salt thereof, according to the invention; in a method of this type, the compound or salt thereof and/or the Factor VII polypeptide may be present in a liquid, aqueous medium.

- the use of a pharmaceutical composition according to the invention for the manufacture of a medicament for the treatment of a condition or disorder against which the Factor VII polypeptide in question is effective.

All references, including publications, patent applications and patents, cited herein are hereby incorporated by reference to the same extent as if each reference was individually and specifically indicated to be incorporated by reference and was set forth in its entirety herein.
All headings and sub-headings are used herein for convenience only and should not be construed as limiting the invention in any way,
Any combination of the above-described elements in all possible variations thereof is encompassed by the invention

unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (for instance all exact exemplary values provided with respect to a particular factor or measurement can be considered to also provide a corresponding approximate measurement, modified by "about," where appropriate).

All methods described herein can be performed in any suitable order unless otherwise indicated herein or otherwise clearly contradicted by context.

The use of any and all examples, or exemplary language ("for instance", "such as") provided herein, is intended merely to better illuminate the invention and does not pose a limitation on the scope of the invention unless otherwise indicated.

No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents,

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (for instance a composition described herein as comprising a particular element should be understood as also describing a composition consisting of that element, unless otherwise stated or clearly contradicted by context).

This invention includes all modifications and equivalents of the subject matter recited in the aspects or claims presented herein to the maximum extent permitted by applicable law.

## EXAMPLES

*General methods*

The following abbreviations are used:

| | |
|---|---|
| Ac: | acetyl |
| Boc: | tert-butyloxycarbonyl |
| DCM: | dichloromethane, methylenechloride |
| DIPEA | diisopropylethylamine |
| DMF: | N,N-dimethyl formamide |
| DMSO: | dimethyl sulfoxide |
| EDAC: | N-ethyl-N'-(3-dimethylaminopropyl)carbodiimide hydrochloride |
| ELS: | evaporative light scattering |
| Et: | ethyl |
| HOBt: | N-hydroxybenzotriazole, 1-hydroxybenzotriazole |
| Me: | methyl |
| NMP: | N-methylpyrrolidone |
| HPLC: | high pressure liquid chromatography |
| LCMS: | liquid chromatography coupled with mass spectrometry |
| r.t. | room temperature |
| Su: | succinimidyl |
| TFA: | trifluoroacetic acid |

<u>Assay procedures</u>

*(1) Enzyme Inhibition - general*

The ability of compounds of formula I to inhibit Factor VIIa, or other enzymes/Factors such as, e.g., Factor Xa, thrombin, plasmin or trypsin, is assessed by determining the concentration of the compound of formula I that inhibits the activity of the enzyme/Factor in question by 50%, i.e. the $IC_{50}$ value, which is related to the inhibition constant Ki. Purified enzymes are used in chromogenic assays. The concentration of inhibitor (compound of formula I) that causes a 50%

decrease in the rate of hydrolysis of an appropriate substrate is determined by linear regression after plotting the relative rates of hydrolysis (compared to the uninhibited control) versus the log of the concentration of the compound of formula I.

For calculating the inhibition constant Ki, correction of the $IC_{50}$ value for competition with substrate is taken account of by using the formula:

$$Ki = IC_{50}/\{1+(\text{substrate concentration}/Km)\},$$

where Km is the Michaelis-Menten constant [Chen and Prusoff, Biochem. Pharmacol. 22 (1973), 3099-3108; I. H. Segal, Enzyme Kinetics, 1975, John Wiley & Sons, New York, 100-125; both of which references are incorporated herein in their entirety by reference].

(1)a *Factor VIIa (FVIIa) assay*

The inhibitory activity [expressed as inhibition constant Ki(FVIIa)] of compounds of formula I towards Factor VIIa/tissue factor activity may be determined using a chromogenic assay essentially as described previously [J. A. Ostrem et al., Biochemistry 37 (1998) 1053-1059, which reference is incorporated herein in its entirety by reference). Kinetic assays are conducted at 25° C. in half-area microtiter plates (Costar Corp., Cambridge, Mass.) using a kinetic plate reader (Molecular Devices Spectramax 250). In a typical assay, 25 μl rhFVIIa and TF (final concentrations 5 nM and 10 nM, respectively) are combined with 40 μl of inhibitor dilutions in 10% DMSO/TBS-PEG buffer (50 mM Tris, 15 mM NaCl, 5 mM $CaCl_2$, 0.05% PEG 8000, pH 8.15). Following a 15 minute preincubation period, the assay is initiated by the addition of 35 μl of the chromogenic substrate S-2288 (D-Ile-Pro-Arg-p-nitroanilide, Pharmacia Hepar Inc., 500 μM final concentration). The compounds of the present invention inhibit FVIIa/TF with $IC_{50}$-values ranging from 3 mM to < 1 μM.

The following assays (1)b-e and (2)a-c may be employed to investigate the possible inhibition of certain other coagulation enzymes and other serine proteases by compounds of formula I, and thus to determine the specificity of compounds of formula I.

(l)b *Factor Xa assay*

A TBS-PEG buffer of composition 50 mM Tris-Cl, pH 7.8, 200 mM NaCl, 0.05% (w/v) PEG-8000, 0.02% (w/v) $NaN_3$) is used for this assay. The $IC_{50}$ is determined by combining:

25 μl human factor Xa (Enzyme Research Laboratories, Inc.; South Bend, Ind.) in TBS-PEG buffer;
40 μl 10% (v/v) DMSO in TBS-PEG buffer (uninhibited control) or various concentrations of the compound to be tested diluted in 10% (v/v) DMSO in TBS-PEG; and substrate S-2765 [N(α)-benzyloxycarbonyl-D-Arg-Gly-L-Arg-p-nitroanilide; Kabi Pharmacia, Inc.; Franklin, Ohio] in TBS-PEG, in appropriate wells of a Costar half-area microtiter plate.

The assay is performed by pre-incubating the compound of formula I plus enzyme for 10 min. The assay is then initiated by adding substrate to obtain a final volume of 100 μl. The initial velocity of chromogenic substrate hydrolysis is measured by the change in absorbance at 405 nm using a Bio-tek Instruments kinetic plate reader (Ceres UV900HDi) at 25° C. during the linear portion of the time course (usually 1.5 min after addition of substrate). The enzyme concentration is 0.5 nM, and substrate concentration is 140 μM.

(1)c *Thrombin assay*

TBS-PEG buffer is likewise used in this assay. The $IC_{50}$ is determined as above for the Factor Xa assay, except that the substrate employed is S-2366 (L-PyroGlu-L-Pro-L-Arg-p-nitroanilide; Kabi) and the enzyme is human thrombin (Enzyme Research Laboratories, Inc.; South Bend, Ind.). The enzyme concentration is 175 μM.

(1)d *Plasmin assay*

TBS-PEG buffer is likewise used in this assay. The $IC_{50}$ is determined as described above for the Factor Xa assay, except that the substrate employed is S-2251 (D-Val-L-Leu-L-Lys-p-nitroanilide; Kabi) and the enzyme is human plasmin (Kabi). The enzyme concentration is 5 nM and the substrate concentration is 300 μM.

(1)e *Trypsin assay*

TBS-PEG buffer containing 10 mM $CaCl_2$ is used for this assay. The $IC_{50}$ is determined as described above for the Factor Xa assay, except that the substrate employed is BAPNA (benzoyl-L-Arg-p-nitroanilide; Sigma Chemical Co.; St. Louis, Mo.) and the enzyme is bovine pancreatic trypsin (Type XIII, TPCK treated; Sigma). The enzyme concentration is 50 nM and the substrate concentration is 300 μM.

*(2)a Amidolytic assays for FIXa and tPA.*

Enzymes and substrates are from American Diagnostica; FIXa (cat no 449b), FIXa substrate (cat no 299F), tPA (cat no 170) and tPA substrate (cat no 444LF). Hydrolysis of substrates 299F and 444LF is followed in a Spectramax Fluorimeter at 360 nm excitation and 440 nm emission. Hydrolysis of substrate 251 and S-2288 was followed in a Spectramax Spectrophotometer at 405 nm.
All assays are performed in a buffer consisting of 50 mM Hepes pH 7.4, 100 mM NaCl, 5 mM CaCl2, 0.01% Tween80. Inhibitors are used at 10, 20, 50, 100, 200 μM concentration. The FIXa assay is performed using 100 μM substrate, the tPA assay is performed using 10 μM substrate.

*(2)b Amidolytic assays for FXIa and FXIIa*

The enzymes FXIa and FXIIa are from American Diagnostica; FXIa (cat no 4011a), FXIIa (cat no 412HA) and trypsin (cat no 20465) are from Life Technology. The chromogenic substrates (Chromogenix) in use are 2288 for FXIa and 2765 for FXIIa.
Hydrolysis of the chromogenic substrates is followed in a Spectramax Spectrophotometer at 405 nm for 10-20 min with intervals of 5-20 sec depending on the enzyme.
All assays are performed in a buffer consisting of 50 mM Hepes pH 7.4, 100 mM NaCl, 5 mM CaCl2, 0.01% BSA. With the exception that for the FIXa assays ethylene glycol is further added to a final concentration of 40%. In the assays substrate concentrations of 50, 100, 200, 500, 1000 μM are employed for each inhibitor concentration: 25, 50, 100, 500 μM.

*(2)c Data analysis*

For the assays run at a single substrate concentration, KI is determined using the formula V0/VI= 1+ I/KI (valid for S<<Km), by linear fitting of the values determined at several different inhibitor concentrations. V0 is the rate of hydrolysis without inhibitors present, VI is the rate of hydrolysis at the in the presence of inhibitor and I is the inhibitor concentration.
From double reciprocal plots of 1/v versus 1/s for each inhibitor concentration the slope (Km(app)/V) is determined. This is followed by plots of Km(app)/V against the inhibitor concentration. Ki is determined as the intercept of the straight line at the i-axis.

*Preparation of FVII polypeptides*

Human purified Factor VIIa suitable for use in the present invention is preferably made by DNA recombinant technology, e.g. as described by Hagen et al., Proc.Natl.Acad.Sci. USA 83: 2412-2416, 1986 or as described in European Patent No. 200.421 (ZymoGenetics). The Factor VII variants described herein may be produced by means of recombinant nucleic acid techniques. In general, a cloned wild-type Factor VII nucleic acid sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known (see U.S. 4,784,950, where the cloning and expression of recombinant human Factor VII is described). The bovine Factor VII sequence is described in Takeya et al., J. Biol. Chem. 263:14868-14872 (1988)). It will be understood that any conventional technique may be used to produce Factor VIIa sequence variants.
Factor VII may also be produced by the methods described by Broze and Majerus, J. Biol.Chem. 255 (4): 1242-1247, 1980 and Hedner and Kisiel, J. Clin.Invest. 71: 1836-1841, 1983. These methods yield Factor VII without detectable amounts of other blood coagulation Factors. An even further purified Factor VII preparation may be obtained by including an additional gel filtration as the final purification step.
Chemical and/or enzymatic modification of wild-type Factor VIIa or Factor VIIa sequence variants may be achieved by any means known in the art. Suitable modifications include, without limitation, chemical glycan modifications as described in EP application, EP06120000 (Novo Nordisk A/S), C-terminal modifications as described in WO2006013202, glycosyltransferase mediated modification as described in WO2006035057, dendrimer modification as described in WO2005014049, carboxypeptidase mediated terminal modification (as described, e.g., in W02005035553-A2, W09520039-A, and W09838285-A), transglutaminase-mediated modification (as described, e.g., W02005070468), autocatalytic/endopeptidase-mediated transpeptidation (as described, e.g., in W02006013202-A2); thiol-mediated modification (as described, e.g., in WO2002077218 and PCT/EP/2006063310) and amine-mediated modification (as described, e.g., in WO02/02764.

Factor VII and Factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, Factor VII may be activated by passing it through an ion-exchange chromatography column, such as Mono Q® (Pharmacia) or the like. The resulting activated Factor VII may then be formulated and administered as described below.

*Stabilizing effect of a compound of formula (I) on FVIIa*

The biological activity of a FVII polypeptide, such as FVIIa, may be measured using a one-stage coagulation assay. For this purpose, the sample to be tested is diluted in 50 mM PIPES-buffer (pH 7.5), 0.1% BSA, and 40 μl of this solution is incubated with 40 μl of FVII-deficient plasma and 80 μl of human recombinant TF containing 10 mM $Ca^{2+}$ and synthetic phospholipids. Coagulation times are measured and compared to a standard curve using a reference standard in a parallel line assay.

*Assays suitable for determining biological activity of Factor VII polypeptides*

Factor VII polypeptides useful in accordance with the present invention may be selected by suitable assays that can be performed as simple preliminary in vitro tests. Thus, the present specification discloses a simple test (entitled "In Vitro Hydrolysis Assay") for the activity of Factor VII polypeptides.

<u>1st generation clot assay</u>

The activity of the Factor VII polypeptides may be measured using a one-stage clot assay essentially as described in WO 92/15686 or US 5,997,864. Briefly, the sample to be tested is diluted in 50 mM Tris (pH 7.5), 0.1% BSA and 100 μL is incubated with 100 μL of Factor VII deficient plasma and 200 μL of thromboplastin C containing 10 mM Ca2+. Clotting times are measured and compared to a standard curve using a reference standard or a pool of citrated normal human plasma in serial dilution.

<u>In Vitro Hydrolysis Assay (Assay 1)</u>

Native (wild-type) Factor VIIa and Factor VII polypeptide (both hereinafter referred to as "Factor VIIa") may be assayed for specific activities. They may also be assayed in parallel to directly compare their specific activities. The assay is carried out in a microtiter plate (MaxiSorp, Nunc, Denmark). The chromogenic substrate D-Ile-Pro-Arg-p-nitroanilide (S-2288, Chromogenix, Sweden), final concentration 1 mM, is added to Factor VIIa (final concentration 100 nM) in 50 mM HEPES, pH 7.4, containing 0.1 M NaCl, 5 mM $CaCl_2$ and 1 mg/mL bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used for calculating the ratio between the activities of Factor VII polypeptide and wild-type Factor VIIa:

$$\text{Ratio} = (\text{A405 nm Factor VII polypeptide})/(\text{A405 nm Factor VIIa wild-type}).$$

Based thereon, Factor VII polypeptides with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

The activity of the Factor VII polypeptides may also be measured using a physiological substrate such as Factor X ("In Vitro Proteolysis Assay"), suitably at a concentration of 100-1000 nM, where the Factor Xa generated is measured after the addition of a suitable chromogenic substrate (eg. S-2765). In addition, the activity assay may be run at physiological temperature.

In Vitro Proteolysis Assay (Assay 2)

Native (wild-type) Factor VIIa and Factor VII polypeptide (both hereinafter referred to as "Factor VIIa") are assayed in parallel to directly compare their specific activities. The assay is carried out in a microtiter plate (MaxiSorp, Nunc, Denmark). Factor VIIa (10 nM) and Factor X (0.8 microM) in 100 μL 50 mM HEPES, pH 7.4, containing 0.1 M NaCl, 5 mM $CaCl_2$ and 1 mg/mL bovine serum albumin, are incubated for 15 min. Factor X cleavage is then stopped by the addition of 50 μL 50 mM HEPES, pH 7.4, containing 0.1 M NaCl, 20 mM EDTA and 1 mg/mL bovine serum albumin. The amount of Factor Xa generated is measured by the addition of the chromogenic substrate Z-D-Arg-Gly-Arg-p-nitroanilide (S-2765, Chromogenix, Sweden), final concentration 0.5 mM. The absorbance at 405 nm is measured continuously in a SpectraMax™ 340 plate reader (Molecular Devices, USA). The absorbance developed during 10 minutes, after subtraction of the absorbance in a blank well containing no FVIIa, is used for calculating the ratio between the proteolytic activities of Factor VII polypeptide and wild-type Factor VIIa:

Ratio = (A405 nm Factor VII polypeptide)/(A405 nm Factor VIIa wild-type).

Based thereon, a Factor VII polypeptide with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

Thrombin generation assay (Assay 3)

The ability of a Factor VII polypeptides to generate thrombin can be measured in an assay (Assay 3) comprising all relevant coagulation Factors and inhibitors at physiological concentrations (minus Factor VIII when mimicking hemophilia A conditions) and activated platelets (as described on p. 543 in Monroe et al. (1997) Brit. J. Haematol. 99, 542-547 which is hereby incorporated as reference).

One-stage Coagulation Assay (Clot Assay) (Assay 4)

Factor VII polypeptides may also be assayed for specific activities ("clot activity") by using a one-stage coagulation assay (Assay 4). For this purpose, the sample to be tested is diluted in 50 mM PIPES-buffer (pH 7.2), 1% BSA and 40 μl is incubated with 40 μl of Factor VII deficient plasma and 80 μl of human recombinant tissue factor containing 10 mM $Ca^{2+}$ and synthetic phospholipids. Coagulation times (clotting times) are measured and compared to a standard curve using a reference standard in a parallel line assay.

*Determination of content of heavy chain degradation product, oxidized forms, and aggregates in FVII polypeptides*

Content of oxidized forms and heavy chain degradation products is determined by RP-HPLC as described in the following (Assay 5): Reverse phase HPLC was run on a proprietary 4.5x250 mm butyl-bonded silica column with a particle size of 5 μm and pore size 300Å. Column temperature: 70°C. A-buffer: 0.1% v/v trifluoracetic acid. B-buffer: 0.09% v/v trifluoracetic acid, 80% v/v acetonitrile. The column was eluted with a linear gradient from X to (X+13)% B in 30 minutes. X was adjusted so that FVIIa elutes with a retention time of approximately 26 minutes. Flow rate: 1.0 mL/min. Detection: 214 nm. Load: 25 μg FVIIa.

The content of aggregates is determined by non-denaturing size exclusion HPLC:

Non-denaturing size exclusion chromatography was run on a Waters Protein Pak 300 SW column, 7,5x300 mm using 0.2 M ammoniumsulfat, 5% 2-propanol pH 7,0 as mobile phase. Flow rate :0.5 ml/min. Detection: 215 nm. Load: 25μg FVIIa.

*NMR analysis*

NMR spectra were recorded on Bruker 300 MHz and 400 MHz instruments. HPLC-MS was performed on a Perkin Elmer instrument (API 100).

*HPLC analysis*

HPLC-systems from Merck-Hitachi (Hibar™ RT 250-4, Lichrosorb™ RP 18, 5.0 μm, 4.0 x 250 mm, gradient elution, 20% to 80% acetonitrile in water within 30 min, 1.0 ml/min, detection at 254 nm) and Waters (Symmetry™, C18, 3.5 μm, 3.0 x 150 mm, gradient elution, 5% to 90% acetonitrile in water within 15 min, 1.0 ml/min, detection at 214 nm) were used.

**General method (A)**

The compounds of formula (I) according to this invention may be prepared as follows:

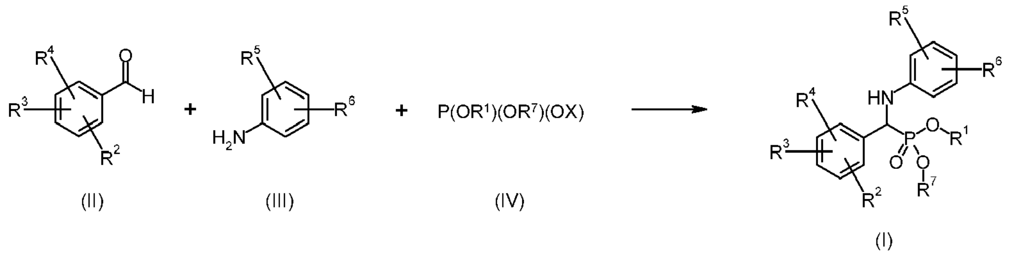

A mixture of an aldehyde of formula (II), an aniline of formula (III), optionally as salt of a strong acid (e.g. as hydrochloride), and a phosphite of formula (IV), wherein X represents hydrogen, lower alkyl, or the same substituent as $R^1$ or $R^7$, in an appropriate solvent such as acetic acid, THF, dioxane, water, acetonitrile, DMF, NMP, or mixtures thereof, is stirred at room temperature or at higher temperatures, until one of the three starting materials is consumed. The product (I) is isolated and purified using conventional techniques, such as crystallization or chromatography.

**General method (B)**

Compounds of general formula (I), in which $R^7$ represents hydrogen, may be prepared as follows:

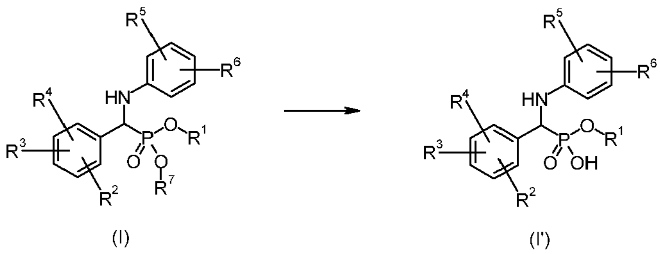

To the product of formula (I), in which $R^7$ is not hydrogen, an aqueous solution of sodium hydroxyde or an equivalent reagent (e.g. KOH, LiOH, CsOH) is added, followed by the optional addition of an organic solvent such as THF or dioxane. The resulting mixture is stirred at room temperature or at higher temperatures until the starting material is consumed. The product (I') is isolated and purified using conventional techniques, such as crystallization or chromatography.

*Working examples*

**Example 1.** [(4-Benzyloxy-3-methoxyphenyl)(4-carbamimidoylphenylamino)methyl]phosphonic acid dimethyl ester hydrochloride

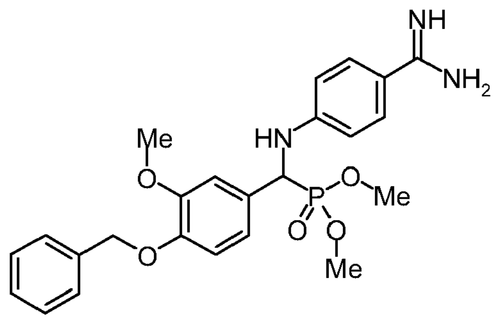

To a mixture of 4-aminobenzamidine dihydrochloride (104 mg, 0.5 mmol), AcOH (2 ml), and 4-benzyloxy-3-methoxybenzaldehyde (124 mg, 0.5 mmol) was added trimethylphosphite (0.18 ml, 1.5 mmol). The mixture was stirred at room temperature for two days, and then diluted with aqueous 1N HCl (10 ml). The resulting solution was kept overnight at 4 °C, and the precipitated solid was isolated by filtration. Drying under reduced pressure yielded 146 mg (58%) of the title compound as colorless solid.
LCMS: $MH^+$ = 470. 94% pure by ELS.

**Example 2.** [(4-Benzyloxy-3-methoxyphenyl)(4-carbamimidoylphenylamino)methyl]phosphonic acid monomethyl ester hydrochloride

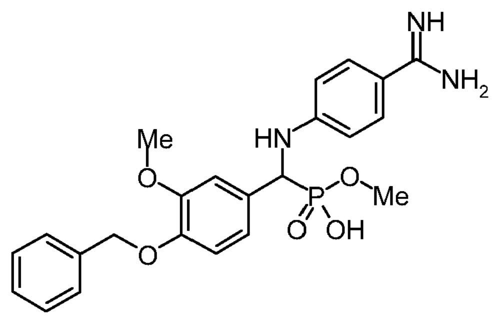

To [(4-benzyloxy-3-methoxyphenyl)(4-carbamimidoylphenylamino)methyl]phosphonic acid dimethyl ester hydrochloride (42 mg, 83 μmol) were added aqueous 1N NaOH (1 ml) and THF (0.5 ml). The resulting mixture was stirred at room temperature for 17 h, diluted with aqueous 1N HCl (2.5 ml), EtOH and toluene, concentrated under reduced pressure, and coevaporated once more with a mixture of EtOH and toluene. The residue was suspended in dry EtOH (2.5 ml), filtered, and the filtrate was concentrated under reduced pressure. The residue was triturated with AcOEt (3 ml), the solvent was decanted off, and the solid residue dried in vacuum. 38 mg (93%) of the title compound was obtained as a solid.
LCMS: $MH^+$ = 456. 98% pure by ELS.

**Example 3.** [(4-Carbamimidoylphenylamino)(3,5-diethoxyphenyl)methyl]phosphonic acid monobenzyl ester hydrochloride

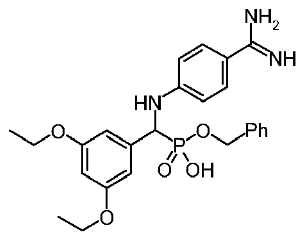

1. 3,5-Diethoxybenzaldehyde

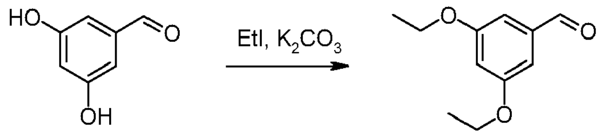


To a solution of 3,5-dihydroxybenzaldehyde (0.64 g, 4.64 mmol) in DMSO (5 ml) were added ethyl iodide (1.25 ml, 15.5 mmol) and $K_2CO_3$ (1.73 g, 12.5 mmol), and the resulting mixture was stirred at 60 °C for 20 h and then at room temperature for 2 d. The mixture was stirred under reduced pressure for 3 h (to remove the excess ethyl iodide) and diluted with water (100 ml) and 1N HCl (20 ml). The product was extracted twice with AcOEt, the combined extracts were washed with brine, dried ($MgSO_4$), and concentrated under reduced pressure. The residual oil (0.98 g) was used in the next reaction without further purification. $^1$H NMR (300 MHz, $CDCl_3$): δ = 1.42 (t, J = 7 Hz, 6H), 4.06 (quart, J = 7 Hz, 4H), 6.69 (t, J = 2 Hz, 1H), 6.98 (d, J = 2 Hz, 2H), 9.86 (s, 1H).

2. [(4-Carbamimidoylphenylamino)(3,5-diethoxyphenyl)methyl]phosphonic acid dibenzyl ester trifluoroacetate

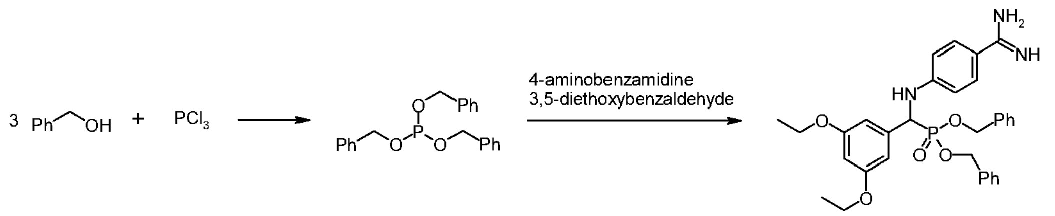


To a mixture of THF (1.5 ml), DIPEA (445 μl, 2.5 mmol), and benzyl alcohol (240 μl, 2.3 mmol) at 0 °C was slowly added $PCl_3$ (66 ul, 0.75 mmol) while stirring energically. After stirring for 20 min at 0 °C, a mixture of 3,5-diethoxybenzaldehyde (101 mg, 0.52 mmol), AcOH (2 ml), and 4-aminobenzamidine dihydrochloride (102 mg, 0.49 mmol) was added at once, and the resulting mixture was stirred at room temperature for 23 h. Aqueous 1N HCl (8 ml) was added, and the solution was kept for 4 h at 4 °C. Decantation yielded an oil, which was purified by preparative HPCL. 68 mg (20%) of the title compound was obtained as an oil.
LCMS: $MH^+$ = 574. 99% pure by ELS.

3. [(4-Carbamimidoylphenylamino)(3,5-diethoxyphenyl)methyl]phosphonic acid monobenzyl ester hydrochloride

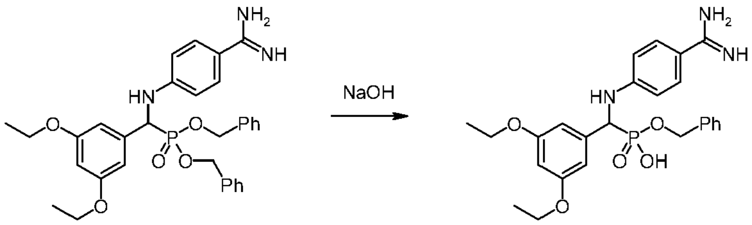


[(4-Carbamimidoylphenylamino)(3,5-diethoxyphenyl)methyl]phosphonic acid dibenzyl ester trifluoroacetate (68 mg, 99 μmol) was mixed with THF (1 ml) and aqueous 1N NaOH (1.5 ml), and the mixture was stirred at room temperature for 18 h. The mixture was diluted with aqueous 1N HCl (3 ml), EtOH, and toluene, concentrated under reduced pressure, and the residue was coevaporated once more with a mixture of EtOH and toluene. The residue was suspended dry EtOH (2.5 ml), filtered, and the filtrate was concentrated under reduce pressure. The remaining solid was triturated with AcOEt (4 ml), filtered off, and dried in vacuum, to yield 16 mg (31%) of the title compound. LCMS: $MH^+ = 484$. 100% pure by ELS.

## Claims

1. A compound of general formula (I)

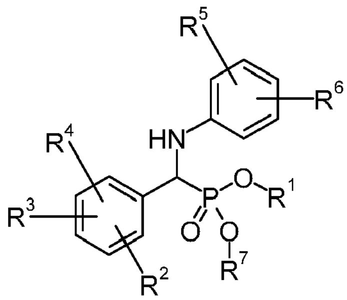

wherein,

$R^1$ represents hydrogen, straight or branched ($C_1$-$C_{15}$) alkyl, (cycloalkyl)alkyl, benzyl, 1-arylethyl, 2-arylethyl, heteroarylmethyl, 1-heteroarylethyl, 2-heteroarylethyl, aryl, or heteroaryl, optionally substituted with halogen, amino, hydroxyl, cyano, $-CONH_2$, nitro, acetyl, cyclopropanoyl, or carboxyl;
$R^2$ represents hydrogen, halogen, methylsulfonylamino, phenylsulfonylamino, or chlorophenylsulfonylamino;
$R^3$ and $R^4$ independently represent methoxy, ethoxy, lower alkoxy, benzyloxy, lower alkyl, halogen, amino, hydroxyl, cyano, $-CONH_2$, nitro, acetyl, cyclopropanoyl, carboxylmethyl, or carboxyl;
$R^5$ represents hydrogen, hydroxyl, or halogen;
$R^6$ represents $-C(=NH)-NH_2$, $-C(=N-OH)-NH_2$, $-C(=NNH_2)-NH_2$, $-NH-C(=NH)-NH_2$, or - $C(=NH)-NHCO_2R^8$, wherein $R^8$ represents lower alkyl, aryl, or arylmethyl, optionally substituted with lower alkyl, alkoxy, or halogen; and
$R^7$ represents hydrogen, lower alkyl, benzyl, $C(R^9R^{10})OR^{11}$, or $C(=O)OR^{12}$; wherein
$R^9$ and $R^{10}$ independently represent hydrogen, lower alkyl, or aryl;
$R^{11}$ and $R^{12}$ independently represent lower alkyl, aryl, or arylmethyl.
including any and all stereoisomeric form or forms thereof;
and any mixture of two or more such compounds of formula I in any ratio;
and physiologically tolerable salts and prodrugs thereof.

2. A compound according to claim 1, in which $R^1$ represents straight or branched ($C_1$-$C_{15}$) alkyl, (cycloalkyl)alkyl, benzyl, 1-arylethyl, 2-arylethyl, heteroarylmethyl, 1-heteroarylethyl, or 2-heteroarylethyl, wherein said groups are optionally substituted with halogen, amino, or hydroxyl.

3. A compound according to claim 1 or claim 2, in which $R^1$ represents methyl, ethyl, propyl, butyl, isopropyl, or benzyl, wherein said groups are optionally substituted with halogen, amino, or hydroxyl.

4. A compound according to any one of the preceding claims, in which $R^1$ represents methyl or benzyl.

5. A compound according to any one of the preceding claims, in which $R^1$ represents methyl, ethyl or benzyl, and $R^7$ represents hydrogen.

6. A compound according to any one of the preceding claims, in which $R^3$ and $R^4$ independently represent methoxy, ethoxy, lower alkoxy, benzyloxy, lower alkyl, halogen, or carboxyl.

7. A compound according to any one of the preceding claims, in which $R^2$ represents hydrogen, methylsulfonylamino, phenylsulfonylamino, or halogen; and $R^3$ and $R^4$ independently represent methoxy, ethoxy, lower alkoxy, benzyloxy, lower alkyl, or carboxyl.

8. A compound according to any one of the preceding claims, in which $R^2$ represents hydrogen or halogen; and $R^3$ and $R^4$ independently represent methoxy, ethoxy, isopropoxy, or benzyloxy.

9. A compound according to any one of the preceding claims, in which $R^2$ represents hydrogen; and $R^3$ and $R^4$ represent 3-methoxy and 4-benzyloxy.

10. A compound according to any one of the preceding claims, in which $R^2$ represents hydrogen or fluorine; and $R^3$ and $R^4$ represent 3,5-diethoxy.

11. A compound according to any one of the preceding claims, in which $R^5$ represents hydrogen or hydroxyl.

12. A compound according to any one of the preceding claims, in which $R^5$ represents hydrogen or hydroxyl, and $R^6$ represents $-C(=NH)-NH_2$.

13. A compound according to any one of the preceding claims, in which $R^5$ represents hydrogen, and $R^6$ represents 4-$C(=NH)-NH_2$.

14. A compound according to any one of claims 1-13, wherein the compound is selected from the list of:

[(4-benzyloxy-3-methoxyphenyl)(4-carbamimidoylphenylamino)methyl]phosphonic acid dimethyl ester;
[(4-benzyloxy-3-methoxyphenyl)(4-carbamimidoylphenylamino)methyl]phosphonic acid monomethyl ester;
[(4-benzyloxy-3-methoxyphenyl)(4-carbamimidoylphenylamino)methyl]phosphonic acid dibenzyl ester;
[(4-benzyloxy-3-methoxyphenyl)(4-carbamimidoylphenylamino)methyl]phosphonic acid monobenzyl ester;
[(4-benzyloxy-3-ethoxyphenyl)(4-carbamimidoylphenylamino)methyl]phosphonic acid dimethyl ester;
[(4-benzyloxy-3-ethoxyphenyl)(4-carbamimidoylphenylamino)methyl]phosphonic acid monomethyl ester;
[(4-benzyloxy-3-ethoxyphenyl)(4-carbamimidoylphenylamino)methyl]phosphonic acid dibenzyl ester;
[(4-benzyloxy-3-ethoxyphenyl)(4-carbamimidoylphenylamino)methyl]phosphonic acid monobenzyl ester;
[(4-isopropoxy-3-ethoxyphenyl)(4-carbamimidoylphenylamino)methyl]phosphonic acid dimethyl ester;
[(4-isopropoxy-3-ethoxyphenyl)(4-carbamimidoylphenylamino)methyl]phosphonic acid monomethyl ester;
[(4-isopropoxy-3-ethoxyphenyl)(4-carbamimidoylphenylamino)methyl]phosphonic acid dibenzyl ester;
[(4-isopropoxy-3-ethoxyphenyl)(4-carbamimidoylphenylamino)methyl]phosphonic acid monobenzyl ester;
[(4-benzyloxy-3-ethoxy-6-methylsulfonylaminophenyl)(4-carbamimidoylphenylamino)methyl]phosphonic acid dimethyl ester;
[(4-benzyloxy-3-ethoxy-6-methylsulfonylaminophenyl)(4-carbamimidoylphenylamino)methyl]phosphonic acid monomethyl ester;
[(4-benzyloxy-3-ethoxy-6-methylsulfonylaminophenyl)(4-carbamimidoylphenylamino)methyl]phosphonic acid dibenzyl ester;
[(4-benzyloxy-3-ethoxy-6-methylsulfonylaminophenyl)(4-carbamimidoylphenylamino)methyl]phosphonic acid monobenzyl ester; [(3,4-diethoxyphenyl)(4-carbamimidoylphenylamino)methyl]phosphonic acid dimethyl ester;
[(3,4-diethoxyphenyl)(4-carbamimidoylphenylamino)methyl]phosphonic acid monomethyl ester;
[(3,4-diethoxyphenyl)(4-carbamimidoylphenylamino)methyl]phosphonic acid dibenzyl ester;
[(3,4-diethoxyphenyl)(4-carbamimidoylphenylamino)methyl]phosphonic acid monobenzyl ester;
[(3,5-diethoxyphenyl)(4-carbamimidoylphenylamino)methyl]phosphonic acid dimethyl ester;

[(3,5-diethoxyphenyl)(4-carbamimidoylphenylamino)methyl]phosphonic acid monomethyl ester;
[(3,5-diethoxyphenyl)(4-carbamimidoylphenylamino)methyl]phosphonic acid dibenzyl ester;
[(3,5-diethoxyphenyl)(4-carbamimidoylphenylamino)methyl]phosphonic acid monobenzyl ester;
including any and all stereoisomeric form or forms thereof;
and any mixture of two or more such compounds of formula I in any ratio;
and physiologically tolerable salts and prodrugs thereof.

**15.** A compound according to claim 14, wherein the compound is selected from the list of:

[(4-benzyloxy-3-methoxyphenyl)(4-carbamimidoylphenylamino)methyl]phosphonic acid dimethyl ester;
[(4-benzyloxy-3-methoxyphenyl)(4-carbamimidoylphenylamino)methyl]phosphonic acid monomethyl ester;
[(4-benzyloxy-3-methoxyphenyl)(4-carbamimidoylphenylamino)methyl]phosphonic acid dibenzyl ester;
[(4-benzyloxy-3-methoxyphenyl)(4-carbamimidoylphenylamino)methyl]phosphonic acid monobenzyl ester;
[(3,5-diethoxyphenyl)(4-carbamimidoylphenylamino)methyl]phosphonic acid dimethyl ester;
[(3,5-diethoxyphenyl)(4-carbamimidoylphenylamino)methyl]phosphonic acid monomethyl ester;
[(3,5-diethoxyphenyl)(4-carbamimidoylphenylamino)methyl]phosphonic acid dibenzyl ester;
[(3,5-diethoxyphenyl)(4-carbamimidoylphenylamino)methyl]phosphonic acid monobenzyl ester;
including any and all stereoisomeric form or forms thereof;
and any mixture of two or more such compounds of formula I in any ratio;
and physiologically tolerable salts and prodrugs thereof.

**16.** A pharmaceutical composition, comprising: one or more compounds according to any one of claims 1-15, or physiologically tolerable salts thereof; and a Factor VII polypeptide.

**17.** A pharmaceutical composition according to claim 22, wherein said Factor VII polypeptide is selected from: wild-type human Factor VIIa; a Factor VII variant; and a Factor VII derivative.

**18.** A pharmaceutical composition according to claim 16 or 17, further comprising a pharmaceutically tolerable carrier or diluent.

**19.** A pharmaceutical composition according to any one of claims 16-18, which is a liquid, aqueous composition.

**20.** A method of preparing a composition, comprising a Factor VII polypeptide, comprising: Adding a compound according to any one of claims 1-15, or a physiologically tolerable salt thereof, to a sample containing said Factor VII polypeptide; or adding said Factor VII polypeptide to a sample containing a compound according to any one of claims 1-15, or a physiologically tolerable salt thereof.

**21.** A method according to claim 20, wherein said Factor VII polypeptide is selected from: wild-type human Factor VIIa; a Factor VII variant; and a Factor VII derivative.

**22.** A method according to claim 20 or 21, wherein said compound or salt thereof and/or said Factor VII polypeptide is present in a liquid, aqueous medium.

**23.** A pharmaceutical composition prepared by a method according to any one of claims 20-22.

**24.** A method of inhibiting a Factor VII polypeptide, comprising: Adding a compound according to any one of claims 1-15, or a physiologically tolerable salt thereof, to a sample containing said Factor VII polypeptide; or adding said Factor VII polypeptide to a sample containing a compound according to any one of claims 1-15, or a physiologically tolerable salt thereof.

**25.** A method according to claim 24, wherein said Factor VII polypeptide is selected from: Wild-type human Factor VIIa; a Factor VII variant; and a Factor VII derivative.

**26.** A method according to claim 24 or 25, wherein said compound or salt thereof and/or said Factor VII polypeptide is present in a liquid, aqueous medium.

**27.** Use of a pharmaceutical composition according to any one of claims 16-19 or 24 for the manufacture of a medicament for the treatment of a condition or disorder against which said Factor VII polypeptide is effective.

**28.** Use of a compound according to any one of claims 1-15, in combination with a Factor VII polypeptide, for the manufacture of a medicament for treatment of a condition or disorder against which said Factor VII polypeptide is effective.

**29.** The use of a compound according to any one of claims 1-15 for the stabilization of a Factor VII polypeptide.

European Patent Office

# EUROPEAN SEARCH REPORT

Application Number
EP 07 10 3677

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| X | GROEBKE ZBINDEN K ET AL: "Design of selective phenylglycine amide tissue factor/factor VIIa inhibitors" BIOORGANIC & MEDICINAL CHEMISTRY LETTERS, vol. 15, no. 3, 1 February 2005 (2005-02-01), pages 817-822, XP004739706 ISSN: 0960-894X * the whole document * ----- | 1-29 | INV. C07F9/40 A61K31/662 A61P7/02 |
| X | GROEBKE ZBINDEN ET AL: "Dose-dependent antithrombotic activity of an orally active tissue factor/factor VIIa inhibitor without concomitant enhancement of bleeding propensity" BIOORGANIC & MEDICINAL CHEMISTRY, vol. 14, no. 15, 1 August 2006 (2006-08-01), pages 5357-5369, XP005508675 ISSN: 0968-0896 * the whole document * ----- | 1-29 | |
| | | | TECHNICAL FIELDS SEARCHED (IPC) |
| X | WO 01/90051 A (HOFFMANN LA ROCHE) 29 November 2001 (2001-11-29) * the whole document * ----- | 1-29 | C07F A61K |
| X | WO 00/35858 A (HOFFMANN LA ROCHE) 22 June 2000 (2000-06-22) * the whole document * ----- | 1-29 | |
| X | US 6 140 353 A (ACKERMANN ET AL) 31 October 2000 (2000-10-31) * the whole document * ----- | 1-29 | |
| A | EP 0 703 239 A (HOECHST AG) 27 March 1996 (1996-03-27) * page 24; compound 74 * ----- | 1-29 | |

The present search report has been drawn up for all claims

| Place of search | Date of completion of the search | Examiner |
|---|---|---|
| Munich | 13 September 2007 | Elliott, Adrian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 07 10 3677

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2007

| Patent document cited in search report | Publication date | Patent family member(s) | Publication date |
|---|---|---|---|
| WO 0190051 A | 29-11-2001 | AR 028596 A1 | 14-05-2003 |
| | | AU 8177401 A | 03-12-2001 |
| | | BR 0110998 A | 08-04-2003 |
| | | CA 2408602 A1 | 29-11-2001 |
| | | CN 1430599 A | 16-07-2003 |
| | | HU 0302073 A2 | 29-09-2003 |
| | | JP 3842132 B2 | 08-11-2006 |
| | | JP 2003534311 T | 18-11-2003 |
| | | MA 26905 A1 | 20-12-2004 |
| | | MX PA02011466 A | 25-04-2003 |
| | | NO 20025590 A | 21-11-2002 |
| | | NZ 522412 A | 28-05-2004 |
| | | PL 361355 A1 | 04-10-2004 |
| | | US 2002004608 A1 | 10-01-2002 |
| | | YU 83602 A | 25-05-2006 |
| | | ZA 200208899 A | 19-02-2004 |
| WO 0035858 A | 22-06-2000 | AR 029740 A1 | 16-07-2003 |
| | | AT 274491 T | 15-09-2004 |
| | | AU 758229 B2 | 20-03-2003 |
| | | AU 1862700 A | 03-07-2000 |
| | | BR 9916111 A | 04-09-2001 |
| | | CA 2354023 A1 | 22-06-2000 |
| | | CN 1334798 A | 06-02-2002 |
| | | CZ 20012112 A3 | 17-10-2001 |
| | | DE 69919743 D1 | 30-09-2004 |
| | | DE 69919743 T2 | 01-09-2005 |
| | | ES 2230909 T3 | 01-05-2005 |
| | | HR 20010427 A2 | 30-06-2002 |
| | | HU 0104421 A2 | 28-03-2002 |
| | | ID 29066 A | 26-07-2001 |
| | | JP 3676236 B2 | 27-07-2005 |
| | | JP 2002532459 T | 02-10-2002 |
| | | MA 26766 A1 | 20-12-2004 |
| | | NO 20012921 A | 14-06-2001 |
| | | NZ 511927 A | 27-02-2004 |
| | | PL 348436 A1 | 20-05-2002 |
| | | RU 2198871 C1 | 20-02-2003 |
| | | TR 200101744 T2 | 21-12-2001 |
| | | US 6242644 B1 | 05-06-2001 |
| | | ZA 200104034 A | 19-08-2002 |
| US 6140353 A | 31-10-2000 | AT 243192 T | 15-07-2003 |
| | | AU 739769 B2 | 18-10-2001 |
| | | AU 9521098 A | 24-06-1999 |
| | | CA 2255180 A1 | 04-06-1999 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 07 10 3677

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2007

| Patent document cited in search report | Publication date | Patent family member(s) | Publication date |
|---|---|---|---|
| US 6140353 A | | CN 1224714 A | 04-08-1999 |
| | | CZ 9803969 A3 | 17-11-1999 |
| | | DE 59808751 D1 | 24-07-2003 |
| | | DK 921116 T3 | 06-10-2003 |
| | | ES 2201396 T3 | 16-03-2004 |
| | | HK 1020941 A1 | 07-11-2003 |
| | | HU 9802808 A2 | 28-06-1999 |
| | | IL 127361 A | 12-09-2002 |
| | | JP 3236267 B2 | 10-12-2001 |
| | | JP 11246507 A | 14-09-1999 |
| | | MA 26572 A1 | 20-12-2004 |
| | | NO 985646 A | 07-06-1999 |
| | | NZ 333126 A | 23-06-2000 |
| | | PT 921116 T | 28-11-2003 |
| | | TW 544446 B | 01-08-2003 |
| EP 0703239 A | 27-03-1996 | AU 3068695 A | 04-04-1996 |
| | | CA 2158517 A1 | 20-03-1996 |
| | | DE 4433244 A1 | 28-03-1996 |
| | | JP 8104694 A | 23-04-1996 |
| | | ZA 9507674 A | 15-04-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005016365 A
- US 4784950 A
- WO 0331464 A
- US 20040043446 A
- US 20040063911 A
- US 20040142856 A
- US 20040137557 A
- US 20040132640 A
- WO 2007022512 A
- WO 0104287 A
- US 20030165996 A
- WO 0158935 A
- WO 0393465 A
- WO 0202764 A
- US 20030211094 A
- US 5580560 A
- DK 0200189 W
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- US 6806063 B
- US 20030096338 A
- WO 04029091 A
- WO 04083361 A
- WO 04111242 A
- WO 04108763 A
- WO 0183725 A
- WO 0222776 A
- WO 03027147 A
- WO 04029090 A
- WO 05075635 A
- EP 05108713 A
- JP 2001061479 B
- US 5997864 A
- US 4837028 A
- US 4501728 A
- US 4975282 A
- EP 200421 A
- EP 06120000 A
- WO 2006013202 A
- WO 2006035057 A
- WO 2005014049 A
- WO 2005035553 A2
- WO 9520039 A
- WO 9838285 A
- WO 2005070468 A
- WO 2006013202 A2
- WO 2002077218 A
- EP 2006063310 W
- US 4456591 A
- WO 9215686 A


### Non-patent literature cited in the description


- **MANNING et al.** *Pharmaceutical Research,* 1989, vol. 6, 903-918
- *J. Pharm. Sci.,* 1977, vol. 66, 2
- **LINO et al.** *Arch. Biochem. Biophys.,* 1998, vol. 352, 182-192
- **MOLLERUP et al.** *Biotechnol. Bioeng.,* 1995, vol. 48, 501-505
- **KORNFELT et al.** *Arch. Biochem. Biophys.,* 1999, vol. 363, 43-54
- **PERSSON et al.** *J. Biol. Chem.,* 1997, vol. 272, 19919-19924
- **PERSSON.** *FEBS Letts.,* 1997, vol. 413, 359-363
- Remington's Pharmaceutical Sciences. Mack Publishing Company, 1990
- **CHEN ; PRUSOFF.** *Biochem. Pharmacol.,* 1973, vol. 22, 3099-3108
- **I. H. SEGAL.** Enzyme Kinetics. John Wiley & Sons, 1975, 100-125
- **J. A. OSTREM et al.** *Biochemistry,* 1998, vol. 37, 1053-1059
- **HAGEN et al.** *Proc.Natl.Acad.Sci. USA,* 1986, vol. 83, 2412-2416
- **TAKEYA et al.** *J. Biol. Chem.,* 1988, vol. 263, 14868-14872
- **BROZE ; MAJERUS.** *J. Biol.Chem.,* 1980, vol. 255 (4), 1242-1247
- **HEDNER ; KISIEL.** *J. Clin.Invest.,* 1983, vol. 71, 1836-1841

- **OSTERUD et al.** *Biochem.,* 1972, vol. 11, 2853
- **HEDNER et al.** *J. Clin. Invest.,* 1983, vol. 71, 1836
- **MONROE et al.** *Brit. J. Haematol.,* 1997, vol. 99, 542-547